# EUROPEAN PATENT SPECIFICATION

(11) **EP 4 298 245 B1**
(45) Date of publication and mention of the grant of the patent: **09.09.2026**
(21) Application number: 22760264.6
(22) Date of filing: 22.02.2022
(51) Int. Cl.: C12Q 1/6834, C12Q 1/6837, G01N 33/50, C08G 83/00, C12Q 1/6804, G01N 33/543, G01N 33/68

(54) **STRUCTURE AND METHODS FOR DETECTION OF SAMPLE ANALYTES**
STRUKTUR UND VERFAHREN ZUM NACHWEIS VON PROBENANALYTEN
STRUCTURE ET PROCÉDÉS DE DÉTECTION D'ANALYTES DANS DES ÉCHANTILLONS

(30) Priority: 24.02.2021 US 202163153258 P
(43) Date of publication of application: 03.01.2024
(73) Proprietor: Nautilus Subsidiary, Inc., Seattle WA 98102 (US)
(72) Inventor: GOPINATH, Ashwin, Boulder, Colorado 80301 (US); ROTHEMUND, Paul, Boulder, Colorado 80301 (US); SHETTY, Rishabh, Boulder, Colorado 80301 (US); BOWEN, Shane, Boulder, Colorado 80301 (US); GALIMIDI, Rachel, Boulder, Colorado 80301 (US)
(74) Representative: HGF
(86) International application number: PCT/US2022/017256
(87) International publication number: WO 2022/182635

(56) References cited:
- WO-A1-2016/011364
- WO-A1-2022/060728
- WO-A1-2022/182643
- WO-A2-2020/123316
- US-A1- 2008 268 450
- US-A1- 2020 025 757
- JUN WANG ET AL: "Quantitating Cell-Cell Interaction Functions with Applications to Glioblastoma Multiforme Cancer Cells", NANO LETTERS, vol. 12, no. 12, 6 November 2012 (2012-11-06), pages 6101 - 6106, XP055207349, ISSN: 1530-6984, DOI: 10.1021/nl302748q

## Description

### BACKGROUND

The current state of personalized healthcare is overwhelmingly genome-centric, predominantly focused on quantifying the genes present within an individual. While such an approach has proven to be extremely powerful, it does not provide a clinician with the complete picture of an individual's health. This is because genes are the "blueprints" of an individual and it merely informs the likelihood of developing an ailment. Within an individual these "blueprints" first need to be transcribed into RNA and then translated into various protein molecules, the real "actors" in the cell, in order to have any effect on the health of an individual.

The concentration of proteins, the interaction between the proteins (protein-protein interactions or PPI), as well as the interaction between proteins and small molecules, are intricately linked to the health of different organs, homeostatic regulatory mechanism as well as the interaction of these systems with the external environment. Hence, quantitative information about proteins as well as PPIs is vital to create a complete picture of an individual's health at a given time point as well as to predict any emerging health issues. For instance, the amount of stress experienced by cardiac muscles (e.g. during a heart attack) can be inferred by measuring the concentration of troponin I/II and myosin light chain present within peripheral blood. Similar protein biomarkers have also been identified, validated and are deployed for a wide variety of organ dysfunctions (e.g. liver disease and thyroid disorders), specific cancers (e.g. colorectal or prostate cancer), and infectious diseases (e.g. HIV and Zika). The interaction between these proteins are also essential for drug development and are increasingly becoming a highly sought-after dataset. The ability to detect and quantify proteins and other molecules, within a given sample of bodily fluids, is an integral component of such healthcare development.

Jun Wang ET AL: "Quantitating Cell-Cell Interaction Functions with Applications to Glioblastoma Multiforme Cancer Cells", Nano Letters, vol. 12, no. 12, 6 November 2012, pages 6101-6106 discloses a method for quantitating the distance dependence of cell-cell interactions by employing a microchip design that permits a multiplex, quantitative protein assay from statistical numbers of cell pairs, as a function of cell separation, with a 0.15 nL volume microchamber.

WO 2016/011364 A1 discloses proximity coupling and sequencing methods to screen, identify, validate and/or characterize interactions between analytes and binding moieties as well as to determine or quantify levels of target analytes.

### SUMMARY

The present disclosure generally relates to systems, structures and methods for detection and quantification of analyte molecules in a sample.

The presently claimed invention is as defined in the claims.

The present invention provides a system for detecting analyte molecules in a sample, comprising:
(a) a substrate comprising a plurality of binding locations, each configured to receive one of the supramolecular structures;
(b) a plurality of supramolecular structures, wherein each supramolecular structure comprises:
   i. at least one capture molecule configured to bind to an analyte molecule,
   ii. a core structure comprising a plurality of core molecules, wherein the at least one capture molecule is linked to the core structure through a capture barcode, and
   iii. the capture barcode linked to the core structure at a first location, that can be used to map the location of each respective supramolecular structure on the substrate;
(c) the sample comprising the one or more analytes, wherein upon contacting the sample with the substrate, the one or more analyte molecules interact with a corresponding capture molecule of the plurality of capture molecules;
(d) a signal generation system configured to generate a signal for each analyte molecule bound to a corresponding capture molecule; and
(e) a detection system configured to detect the generated signal;
wherein the locations of the supramolecular structures received on the plurality of binding locations are configured to be mapped using the capture barcodes prior to contacting the supramolecular structures with the sample, thereby allowing creation of a map of unique supramolecular structure locations on the substrate.

In one embodiment, each capture barcode is configured to link with one of the capture molecules.

In one embodiment, the capture molecules are configured to interact with different types of analyte molecules.

In one embodiment, the capture molecules are each configured to interact with a particular analyte molecule.

In one embodiment, the supramolecular structures are DNA origami structures, and the capture molecules are attached to the DNA origami structures through DNA hybridization.

In one embodiment, the supramolecular structure comprises a core structure comprising a scaffolded DNA origami structure.

The present invention provides a method for detecting analyte molecules present in a sample, the method comprising:
providing a plurality of supramolecular structures bound to a substrate at binding locations, each supramolecular structure comprising:
   i. at least one capture molecule configured to bind to an analyte molecule;
   ii. a core structure comprising a plurality of core molecules, wherein the at least one capture molecule is linked to the core structure through a capture barcode, and
   iii. the capture barcode linked to the core structure at a first location;
mapping the location of the plurality of supramolecular structures on the substrate prior to contacting the supramolecular structures with the sample and thereby creating a map of unique supramolecular structure locations on the substrate;
contacting the plurality of supramolecular structures with the sample, such that one or more capture molecules of the plurality of supramolecular structures interacts with a corresponding analyte molecule of the one or more analyte molecules;
generating a signal for each analyte molecule bound to a corresponding capture molecule; and
detecting the signals and thereby detecting the analyte molecules present in the sample;
wherein a capture barcode is linked with each supramolecular structure, and the supramolecular structures on the substrate are mapped via the capture barcodes.

In one embodiment, each capture barcode is configured to link with one of the capture molecules.

In one embodiment, mapping the supramolecular structures on the substrate is performed when the substrate is manufactured.

In one embodiment, further comprising immobilizing the capture molecules on the substrate, and wherein mapping the supramolecular structures on the substrate is performed after the capture molecules have been immobilized on the substrate.

In one embodiment, the capture molecules are configured to interact with different types of analyte molecules.

In one embodiment, the capture molecules are each configured to interact with the same type of analyte molecule.

In one embodiment, further comprising quantifying concentrations of the analyte molecules present in the sample through registration of the mapped capture molecule locations with the detected signals.

In one embodiment, the supramolecular structures comprises a core structure comprising a scaffolded DNA origami structures, and the capture molecules are attached to the DNA origami structures through DNA hybridization.

Disclosed herein, is a method for detecting an analyte molecule present in a sample, the method comprising: a) providing a supramolecular structure comprising: i) a core structure comprising a plurality of core molecules, and ii) a capture barcode linked to the core structure at a first location and configured to form a linkage with a capture molecule; b) linking the supramolecular structure with the capture molecule via the capture barcode; c) contacting the supramolecular structure with the sample, such that the analyte molecule interacts with the capture molecule and is bound thereto, thereby shifting the supramolecular structure from a ground state to an excited state; d) generating a signal via the supramolecular structure in the excited state; and e) detecting the analyte molecule based on the signal.

Disclosed herein, is a method for detecting one or more analyte molecules present in a sample, the method comprising: a) providing a plurality of supramolecular structures, each comprising: i) a core structure comprising a plurality of core molecules, and ii) a capture barcode linked to the core structure at a first location; b) linking the plurality of supramolecular structures each with a capture molecule via the corresponding capture barcode; c) contacting the plurality of supramolecular structures with the sample, such that one or more capture molecules of the plurality of supramolecular structures interacts with a corresponding analyte molecule of the one or more analyte molecules, thereby shifting the corresponding supramolecular structure from a ground state to an excited state; d) generating a signal for each supramolecular structure in an excited state; and e) detecting each analyte molecule based on a corresponding signal generated. In some embodiments, providing the plurality of supramolecular structures comprises providing the supramolecular structures as attached to one or more widgets, one or more solid supports, one or more polymer matrices, one or more solid substrate, one or more molecular condensates, or combinations thereof. In some embodiments, each solid substrate of the one or more solid substrates comprises a planar substrate. In some embodiments, each planar substrate comprises a plurality of binding sites each configured to attach thereto a supramolecular structure of the plurality of supramolecular structures. In some embodiments, each binding site attaches with a supramolecular structure via a corresponding anchor molecule linked with the supramolecular structure. In some embodiments, the method further comprises mapping the location of the plurality of supramolecular structures attached to the plurality of binding sites, wherein said mapping is via 1) the corresponding capture barcode, 2) an anchor barcode linked to the supramolecular structure, and/or 3) another barcode linked to the supramolecular structure. In some embodiments, said mapping occurs prior to providing the plurality of supramolecular structure and/or prior to contacting the plurality of molecules with the sample. In some embodiments, said mapping enables the identification of the capture molecule and corresponding analyte molecule configured to link with a corresponding supramolecular structure attached at a corresponding binding location. In some embodiments, two or more supramolecular structures of the plurality of supramolecular structures are configured to form a linkage with the same analyte molecule of the plurality of analyte molecules via the corresponding capture molecule.

In some embodiments, for any method disclosed herein, the method further comprises identifying each analyte molecule detected. In some embodiments, for any method disclosed herein, the method further comprises quantifying the concentration of each analyte molecule detected. In some embodiments, for any method or system disclosed herein, each capture molecule comprises a protein, a peptide, an antibody, an aptamer (RNA and/or DNA), a small DNA molecule, an affinity binder, or a combination thereof. In some embodiments, for any method or system disclosed herein, each aptamer comprises a modified aptamer. In some embodiments, for any method or system disclosed herein, each modified aptamer is configured to interact specifically with a particular type of analyte molecule. In some embodiments, for any method disclosed herein, the method further comprises detecting each analyte molecule based on the signal generated when said analyte molecule is present in the sample at a count of a single molecule or higher. In some embodiments, for any system disclosed herein, the system is configured to detect each analyte molecule based on the signal generated when said analyte molecule is present in the sample at a count of a single molecule or higher. In some embodiments, for any method or system disclosed herein, the sample comprises a complex biological sample and the method provides for single-molecule sensitivity thereby increasing a dynamic range and quantitative capture of a range of molecular concentrations within the complex biological sample. In some embodiments, for any method or system disclosed herein, the one or more analyte molecules comprises a protein, a peptide, a peptide fragment, a lipid, a DNA, a RNA, an organic molecule, an inorganic molecule, complexes thereof, or any combinations thereof. In some embodiments, for any method or system disclosed herein, the signal comprises a fluorescence signal and/or a visual signal. In some embodiments, for any method or system disclosed herein, the visual signal comprises an optical signal, an electrical signal, or both. In some embodiments, for any method or system disclosed herein, the optical signal comprises a microwave signal, an ultraviolet illumination, a visible illumination, a near infrared illumination, scattering of light, or combinations thereof.

In some embodiments, for any method disclosed herein, generating the signal comprises: a) binding each analyte molecule linked with a corresponding supramolecular structure in the excited state with a precursor molecule; and b) tagging each precursor molecule bound with an analyte molecule with a fluorophore and/or a fluorescently labeled molecule, thereby generating the fluorescence signal. In some embodiments, for any method disclosed herein, the precursor molecule comprises a biotin molecule. In some embodiments, for any method disclosed herein, the biotin molecule comprises a NHS-biotin molecule. In some embodiments, for any method disclosed herein, the NHS-biotin molecule comprises an amine reactive NHS-biotin molecule. In some embodiments, for any method disclosed herein, the fluorescently labeled molecule comprises fluorescently labeled streptavidin, fluorescently labeled avidin, or both. In some embodiments, for any method disclosed herein, generating the signal comprising tagging each analyte molecule linked with a corresponding supramolecular structure in the excited state with a dye molecule, thereby generating the fluorescence signal. In some embodiments, for any method disclosed herein, the dye molecule comprises a NHS-dye molecule. In some embodiments, for any method disclosed herein, the detecting each analyte molecule comprises obtaining a fluorescence readout of the generated signal(s) and correlating each corresponding supramolecular structure with the capture molecule and analyte molecule configured to be linked thereto. In some embodiments, for any method disclosed herein, the correlating of each corresponding supramolecular structure is based on the mapping as described herein. In some embodiments, for any method disclosed herein, the detecting comprises obtaining a fluorescence readout using a fluorescent microscope.

In some embodiments, for any method disclosed herein, generating the signal comprises: a) binding each analyte molecule linked with a corresponding supramolecular structure in the excited state with a precursor molecule; and b) linking each precursor molecule bound to an analyte molecule with a molecule or nanoparticle that scatters light, thereby generating the visual signal. In some embodiments, for any method disclosed herein, the precursor molecule comprises a biotin molecule. In some embodiments, for any method disclosed herein, the biotin molecule comprises a NHS-biotin molecule. In some embodiments, for any method disclosed herein, the NHS-biotin molecule comprises an amine reactive NHS-biotin molecule. In some embodiments, for any method disclosed herein, the molecule or nanoparticle that scatters light comprises a streptavidin molecule, an avidin molecule, or both. In some embodiments, for any method disclosed herein, the streptavidin molecule, the avidin molecule, or both, comprises Qdots or metal nanoparticles. In some embodiments, for any method disclosed herein, the visual signal comprises the visualization of the large streptavidin and/or avidin molecules linked with the precursor molecule. In some embodiments, for any method disclosed herein, the detecting each analyte molecule comprises visualizing the interaction between each precursor molecule and molecule or nanoparticle that scatters light, and correlating each corresponding supramolecular structure with the capture molecule and analyte molecule configured to be linked thereto. In some embodiments, for any method disclosed herein, the correlating of each corresponding supramolecular structure is based on the mapping as described herein. In some embodiments, for any method disclosed herein, the detecting comprises using a interferometric scattering microscope.

In some embodiments, for any method disclosed herein, generating the signal comprises linking each analyte molecule linked with a corresponding supramolecular structure in the excited state with a second capture molecule, wherein each corresponding second capture molecule is 1) fluorescently labeled to generate a fluorescence signal, or 2) unlabeled to generate a visual signal via the sandwich formation through the complex formed with the corresponding analyte molecule. In some embodiments, for any method disclosed herein, the detecting each analyte molecule comprises obtaining fluorescence readout of the generated signal(s) and correlating each corresponding supramolecular structure with the capture molecule and analyte molecule configured to be linked thereto. In some embodiments, for any method disclosed herein, the correlating of each corresponding supramolecular structure is based on the mapping as described herein. In some embodiments, for any method disclosed herein, the detecting comprises obtaining a fluorescence readout using a fluorescent microscope. In some embodiments, for any method disclosed herein, the detecting each analyte molecule comprises visualizing the interaction between each analyte molecule and second capture molecule, and correlating each corresponding supramolecular structure with the capture molecule and analyte molecule configured to be linked thereto. In some embodiments, for any method disclosed herein, the correlating of each corresponding supramolecular structure is based on the mapping as described herein. In some embodiments, for any method disclosed herein, the detecting comprises using a interferometric scattering microscope.

Disclosed here, is a system for detecting one or more analyte molecules in a sample, the system comprising: a) a substrate comprising a plurality of binding locations; b) a plurality of supramolecular structures, wherein each binding location of the plurality of binding locations is configured to receive a supramolecular structure of the plurality of supramolecular structures, wherein each supramolecular structure comprises: i) a core structure comprising a plurality of core molecules, and ii) a capture barcode linked to the core structure at a first location; c) a plurality of capture molecules, wherein each capture barcode is configured to link with a capture molecule of the plurality of capture molecules; d) the sample comprising the one or more analyte molecules, wherein upon contacting the sample with the substrate, the one or more analyte molecules interact with a corresponding capture molecule of the plurality of capture molecules, such that the corresponding supramolecular structure shifts from a ground state to an excited state; e) a signal generation system enabling a signal to generate based on a supramolecular structure in an excited state; and f) a detection system configured to detect each analyte molecule linked with a supramolecular structure in an excited state based on the generated signal. In some embodiments, the signal comprises a fluorescence signal, a visual signal, or both. In some embodiments, the detection system comprises a fluorescence microscope and/or iSCAT. In some embodiments, the location of the plurality of supramolecular structures on the plurality of binding location is configured to be mapped.

In some embodiments, for any method or system disclosed herein, each supramolecular structure is a nanostructure. In some embodiments, for any method or system disclosed herein, each core structure is a nanostructure. In some embodiments, for any method or system disclosed herein, the plurality of core molecules for each core structure are arranged into a predefined shape and/or have a prescribed molecular weight. In some embodiments, for any method or system disclosed herein, the pre-defined shape is configured to limit or prevent cross-reactivity with another supramolecular structure. In some embodiments, for any method or system disclosed herein, the plurality of core molecules for each core structure comprises one or more nucleic acid strands, one or more branched nucleic acids, one or more peptides, one or more small molecules, or combinations thereof. In some embodiments, for any method or system disclosed herein, each core structure independently comprises a scaffolded deoxyribonucleic acid (DNA) origami, a scaffolded ribonucleic acid (RNA) origami, a scaffolded hybrid DNA:RNA origami, a single-stranded DNA tile structure, a multi-stranded DNA tile structure, a single-stranded RNA origami, a multi-stranded RNA tile structure, hierarchically composed DNA or RNA origami with multiple scaffolds, a peptide structure, or combinations thereof. In some embodiments, for any method or system disclosed herein, each analyte molecule interacts with the corresponding capture molecule through a chemical bond. In some embodiments, for any method or system disclosed herein, for each supramolecular structure, the capture molecule is linked to the core structure through a capture barcode, wherein the capture barcode comprises a first capture linker, a second capture linker, and a capture bridge disposed between the first and second capture linkers, wherein the first capture linker is bound to a first core linker that is bound to the first location on the core structure, wherein the capture molecule and the second capture linker are linked together through binding to a third capture linker. In some embodiments, for any method or system disclosed herein, the capture bridge comprises a polymer core. In some embodiments, for any method or system disclosed herein, the polymer core of the capture bridge comprises a nucleic acid (DNA or RNA) of specific sequence or a polymer like PEG. In some embodiments, for any method or system disclosed herein, the first core linker, second core linker, first capture linker, second capture linker, third capture linker independently comprises a reactive molecule or DNA sequence domain. In some embodiments, for any method or system disclosed herein, each reactive molecule independently comprises an amine, a thiol, a DBCO, a maleimide, biotin, an azide, an acrydite, a NHS-ester, a single stranded nucleic acid (RNA or DNA) of specific sequence, one or more polymers like PEG or polymerization initiators, or combinations thereof. In some embodiments, for any method or system disclosed herein, the linkage between the capture barcode and 1) the first core linker, and/or 2) the third capture linker comprises a chemical bond. In some embodiments, for any method or system disclosed herein, the chemical bond comprises a covalent bond. In some embodiments, for any method or system disclosed herein, the capture molecule is bound to the third capture linker through a chemical bond. In some embodiments, for any method or system disclosed herein, the capture molecule is covalently bonded to the third capture linker. In some embodiments, for any method or system disclosed herein, each supramolecular structure further comprises an anchor molecule linked to the core structure. In some embodiments, for any method or system disclosed herein, the anchor molecule is linked to the core structure via an anchor barcode, wherein the anchor barcode comprises a first anchor linker, a second anchor linker, and an anchor bridge disposed between the first and second anchor linkers, wherein the first anchor linker is bound to a third core linker that is bound to a second location on the core structure, wherein the anchor molecule is linked to the second anchor linker. In some embodiments, for any method or system disclosed herein, the anchor molecule comprises an amine, a thiol, a DBCO, a maleimide, biotin, an azide, an acrydite, a NHS-ester, a single stranded nucleic acid (RNA or DNA) of specific sequence, one or more polymers like PEG or polymerization initiators, or combinations thereof. In some embodiments, for any method or system disclosed herein, the anchor bridge comprises a polymer core. In some embodiments, for any method or system disclosed herein, the polymer core of the anchor bridge comprises a nucleic acid (DNA or RNA) of specific sequence or a polymer like PEG. In some embodiments, for any method or system disclosed herein, the third core linker, first anchor linker, second anchor linker, and anchor molecule independently comprise an anchor reactive molecule or DNA sequence domain. In some embodiments, for any method or system disclosed herein, each anchor reactive molecule independently comprises an amine, a thiol, a DBCO, a maleimide, biotin, an azide, an acrydite, a NHS-ester, a single stranded nucleic acid (RNA or DNA) of specific sequence, one or more polymers like PEG or polymerization initiators, or combinations thereof. In some embodiments, for any method or system disclosed herein, the anchor molecule is linked to the second anchor linker through a chemical bond. In some embodiments, for any method or system disclosed herein, the anchor molecule is covalently bonded to the second anchor linker. In some embodiments, for any method or system disclosed herein, the first location is situated on a first side of the core structure, and the second location is situated on a second side of the core structure. In some embodiments, for any method or system disclosed herein, the one or more analyte molecules in the sample are detected simultaneously through multiplexing via one or more supramolecular structures that shifted to an excited state. In some embodiments, for any method or system disclosed herein, each core structure of the plurality of supramolecular structures are identical to each other. In some embodiments, for any method or system disclosed herein, each supramolecular structure comprises a prescribed shape, size, molecular weight, or combinations thereof. In some embodiments, for any method or system disclosed herein, each supramolecular structure comprises a plurality of capture and molecules. In some embodiments, for any method or system disclosed herein, each supramolecular structure comprises a prescribed stoichiometry of the capture. In some embodiments, for any method or system disclosed herein, at least one supramolecular structure of the plurality of supramolecular structures is configured to detect a different analyte molecule from the other supramolecular structures. In some embodiments, for any method or system disclosed herein, the sample comprises a biological particle or a biomolecule. In some embodiments, for any method or system disclosed herein, the sample comprises an aqueous solution comprising a protein, a peptide, a fragment of a peptide, a lipid, DNA, RNA, an organic molecule, a viral particle, an exosome, an organelle, or any complexes thereof. In some embodiments, for any method or system disclosed herein, the sample comprises a tissue biopsy, blood, blood plasma, Urine, Saliva, Tear, Cerebrospinal fluid, extracellular fluid, cultures cells, culture media, discarded tissue, plant matter, a synthetic protein, a bacterial and/or viral sample or fungal tissue, or combinations thereof.

In some embodiments, the supramolecular structure comprises a prescribed shape, size, molecular weight, or combinations thereof, so as to reduce or eliminate cross-reactions with another supramolecular structure. In some embodiments, the supramolecular structure comprises a plurality of capture and detector molecules. In some embodiments, the supramolecular structure comprises a prescribed stoichiometry of the capture and detector molecules so as to reduce or eliminate cross-reactions with another supramolecular structure.

In some embodiments, the sample comprises a biological particle or a biomolecule. In some embodiments, the sample comprises an aqueous solution comprising a protein, a peptide, a fragment of a peptide, a lipid, DNA, RNA, an organic molecule, a viral particle, an exosome, an organelle, or any complexes thereof. In some embodiments, the sample comprises a tissue biopsy, blood, blood plasma, Urine, Saliva, Tear, Cerebrospinal fluid, extracellular fluid, cultures cells, culture media, discarded tissue, plant matter, a synthetic protein, a bacterial and/or viral sample or fungal tissue, or combinations thereof.

### BRIEF DESCRIPTION OF THE DRAWINGS

The invention is defined in the claims. Any of the following Figures that do not fall within the scope of the claims do not form part of the claimed invention and are provided for comparative purposes only.

Specific embodiments of the disclosed systems, or methods will now be described with reference to the drawings.
FIG. 1A depicts an exemplary depiction of a supramolecular structure and the related subcomponents.
FIG. 1B depicts the supramolecular structure of FIG. 1A with a capture molecule linked thereto.
FIG. 2 provides an exemplary depiction of a method for detecting and quantifying analyte molecules using a plurality of supramolecular structures attached to a planar substrate.

### DETAILED DESCRIPTION

Throughout this application, various embodiments of this disclosure may be presented in a range format. It should be understood that the description in range format is merely for convenience and brevity.

Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

The terms "about" and "approximately" means within an acceptable error range for the particular value as determined by one of ordinary skill in the art, which will depend in part on how the value is measured or determined, i.e., the limitations of the measurement system. For example, "about" can mean within 1 or more than 1 standard deviation, per the practice in the art. Alternatively, the terms can mean a range of up to 20%, up to 10%, up to 5%, or up to 1% of a given value. Alternatively, the terms can mean within an order of magnitude, preferably within 5-fold, and more preferably within 2-fold, of a value.

As used herein, the term "analytes" and "analyte molecules" are used interchangeably.

As used herein, the terms "binding", "bound", and "interaction" are used interchangeably, and generally refer to a non-covalent interaction between macromolecules (e.g., between a protein and a nucleic acid). While in a state of non-covalent interaction, the macromolecules are said to be "associated" or "interacting" or "binding" (e.g., when a molecule X is said to interact with a molecule Y, it is meant the molecule X binds to molecule Y in a non-covalent manner).

As used herein, the terms "attaching", "linking", "linkage", and "link" are used interchangeably, and generally refer to connecting one entity to another. For example, oligomers and primers may be attached to the surface of a capture site. With respect to attaching mechanisms, methods contemplated include such attachment means as ligating, non-covalent bonding, binding of biotin moieties such as biotinylated primers, amplicons, and probes to streptavidin, etc. A capture molecule may for example be attached directly to a supramolecular structure (e.g., via a covalent bond, a biotin- streptavidin bond, a DNA oligonucleotide linker, or a polymer linker) or indirectly (e.g., via linkage to an anchor strand, e.g., by conjugation or through a linker such as a capture strand).

Performing single molecule analysis assays on microfluidic chips in a multiplexed, high-throughput/parallel fashion is of interest in many commercially realized devices for multiomic characterization of biological samples. A variety of such assays exist in the literature for DNA sequencing and single molecule quantification. Mass spectrometry and other affinity-based methods (including antibody-based measurements) for protein identification and quantification have classically dominated the area of high-content proteomics, but suffer from limitations ranging from technical issues to throughput and cross-reactivity. Protein binding affinity binders, such as modified aptamers, represent a highly multiplexed technique for quantifying the human proteome to unprecedented levels and enabling the discovery of biomarkers for improved diagnostics and therapeutics with high sensitivity and specificity. Examples of modified aptamers include SOMAmers^{®}. SomaScan^{®} assays has been used to identify potential biomarkers in a range of diseases like malignant tumors, cardiovascular dysfunction, and inflammatory conditions. This fast, highly scalable, massively parallel and multiplexed technique is a powerful tool to enable the advancement of personalized diagnostics and therapeutics.

Disclosed herein are systems and methods for detecting and quantifying one or more analyte molecules present in a sample. In some embodiments, the one or more analyte molecules are detected using one or more supramolecular structures and one or more capture molecules linked to the supramolecular structures, wherein each capture molecule is configured to bind with a unique analyte molecule. In some embodiments, the capture molecules each comprise an affinity binder. In some embodiments, each affinity binder comprises an aptamer. In some embodiments, each aptamer comprises a modified aptamer. In some embodiments, the one or more supramolecular structures are specifically designed to minimize cross-reactivity with each other. In some embodiments, the analyte molecules bound to a corresponding capture molecule is configured to be detected through a signal being generated. In some embodiments, the signal comprises a fluorescent signal or a visual signal. In some embodiments, the signal correlates to a labeled analyte molecule. In some embodiments, a plurality of supramolecular structures are provided on an array substrate, wherein the supramolecular structures are barcoded to map the location of each supramolecular structure on the array. In some embodiments the supramolecular structures are barcoded via a capture barcode that provides a linkage to a particular capture molecule, and/or the supramolecular structures are barcoded through other barcodes added thereto. In some embodiments, analyte molecules are detected and/or quantified using the mapped location of the supramolecular structure on the substrate array.

### Sample

In some embodiments, the sample comprises an aqueous solution comprising protein, peptides, peptide fragments, lipids, DNA, RNA, organic molecules, inorganic molecules, complexes thereof, or any combinations thereof. In some embodiments, the analyte molecules in the sample comprise protein, peptides, peptide fragments, lipids, DNA, RNA, organic molecules, inorganic molecules, complexes thereof, or any combinations thereof. In some embodiments, the analyte molecules comprise intact proteins, denatured proteins, partially or fully degraded proteins, peptide fragments, denatured nucleic acids, degraded nucleic acid fragments, complexes thereof, or combinations thereof. In some embodiments, the sample is obtained from tissue, cells, the environment of tissues and/or cells, or combinations thereof. In some embodiments, the sample comprises tissue biopsy, blood, blood plasma, urine, saliva, a tear, cerebrospinal fluid, extracellular fluid, cultures cells, culture media, discarded tissue, plant matter, synthetic proteins, bacterial, viral samples, fungal tissue, or combinations thereof. In some embodiments, the sample is isolated from a primary source such as cells, tissue, bodily fluids (e.g., blood), environmental samples, or combinations thereof, with or without purification. In some embodiments, the cells are lysed using a mechanical process or other cell lysis methods (e.g., lysis buffer). In some embodiments, the sample is filtered using a mechanical process (e.g., centrifugation), micron filtration, chromatography columns, other filtration methods, or combinations thereof. In some embodiments, the sample is treated with one or more enzymes to remove one or more nucleic acids or one or more proteins. In some embodiments, the sample comprises intact proteins, denatured proteins, partially or fully degraded proteins, peptide fragments, denatured nucleic acids or degraded nucleic acid fragments. In some embodiments, the sample is collected from one or more individual persons, one or more animals, one or more plants, or combinations thereof. In some embodiments, the sample is collected from an individual person, animal and/or plant having a disease or disorder that comprises an infectious disease, an immune disorder, a cancer, a genetic disease, a degenerative disease, a lifestyle disease, an injury, a rare disease, an age-related disease, or combinations thereof.

### Supramolecular Structure

In some embodiments, the supramolecular structure is a programmable structure that can spatially organize molecules. In some embodiments, the supramolecular structure is a supramolecular DNA origami structure. In some embodiments, the supramolecular structure comprises a plurality of molecules linked together. In some embodiments, the plurality of molecules of the supramolecular structure interact with at least some of each other. In some embodiments, the supramolecular structure comprises a specific shape. In some embodiments, the supramolecular structure comprises a prescribed molecular weight based on the plurality of molecules of the supramolecular structure. In some embodiments, the supramolecular structure is a nanostructure. In some embodiments the plurality of molecules are linked together through a bond, a chemical bond, a physical attachment, or combinations thereof. In some embodiments, the supramolecular structure comprises a large molecular entity, of specific shape and molecular weight, formed from a well-defined number of smaller molecules interacting specifically with each other. In some embodiments, the structural, chemical, and physical properties of the supramolecular structure are explicitly designed. In some embodiments, the supramolecular structure comprises a plurality of subcomponents that are spaced apart according to a prescribed distance. In some embodiments, at least a portion of the supramolecular structure is rigid. In some embodiments, at least a portion of the supramolecular structure is semi-rigid. In some embodiments, at least a portion of the supramolecular structure is flexible.

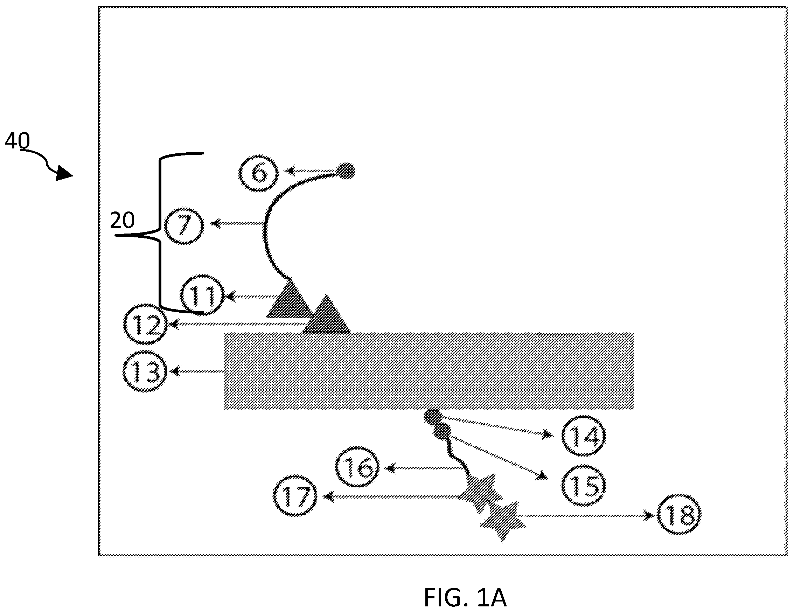

FIG. 1A provides an exemplary embodiment of a supramolecular structure 40 comprising a core structure 13, a capture barcode 20, and an anchor molecule 18. In some embodiments, the supramolecular structure comprises a supramolecular DNA origami structure, wherein the core structure comprises a DNA origami structure. In some embodiments, the supramolecular structure does not comprise an anchor molecule. In some embodiments, the supramolecular structure is a polynucleotide structure.

In some embodiments, the core structure 13 comprises one or more core molecules linked together. In some embodiments, the one or more core molecules comprise 2, 3, 4, 5, 6, 7, 8, 9, 10, 20, 50, 100, 200 or 500 unique molecules that are linked together. In some embodiments, the one or more core molecules comprises from about 2 unique molecules to about 1000 unique molecules. In some embodiments, the one or more core molecules interact with each other and define the specific shape of the supramolecular structure. In some embodiments, the plurality of core molecules interact with each other through reversible non-covalent interactions. In some embodiments, the specific shape of the core structure is a three-dimensional (3D) configuration. In some embodiments, the one or more core molecules provide a specific molecular weight. In some embodiments, the core structure 13 is a nanostructure. In some cases, the one or more core molecules comprise one or more nucleic acid strands (e.g., DNA, RNA, unnatural nucleic acids), one or more branched nucleic acids, one or more peptides, one or more small molecules, or combinations thereof. In some embodiments, the core structure comprises a polynucleotide structure. In some embodiments, at least a portion of the core structure is rigid. In some embodiments, at least a portion of the core structure is semi-rigid. In some embodiments, at least a portion of the core structure is flexible. In some embodiments, the core structure comprises a scaffolded deoxyribonucleic acid (DNA) origami, a scaffolded ribonucleic acid (RNA) origami, a scaffolded hybrid DNA / RNA origami, a single-stranded DNA tile structure, a multi-stranded DNA tile structure, a single-stranded DNA origami, a single-stranded RNA origami, a single-stranded RNA tile structure, a multi-stranded RNA tile structures, a hierarchically composed DNA and/or RNA origami with multiple scaffolds, a peptide structure, or combinations thereof. In some embodiments, the DNA origami is scaffolded. In some embodiments, the RNA origami is scaffolded. In some embodiments, the hybrid DNA/RNA origami is scaffolded. In some embodiments, the core structure comprising a DNA origami, RNA origami, or hybrid DNA/RNA origami that comprises a prescribed two-dimensional (2D) or 3D shape.

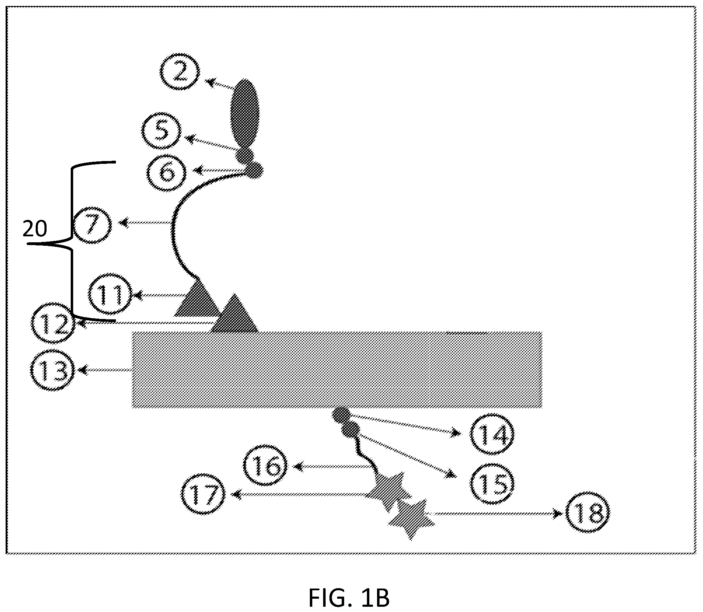

As shown in FIG 1B, in some embodiments, the supramolecular structure is further configured to be linked to a capture molecule 2 via a capture barcode 20, as described herein. In some embodiments, the capture molecule 2 and/or anchor molecule 18 are immobilized with respect to the core nanostructure 13 when linked thereto. In some embodiments, any number of the one or more core molecules comprises one or more core linkers 12,14 configured to form a linkage with a capture molecule 2 and/or an anchor molecule 18. In some embodiments, any number of the one or more core molecules are configured to be linked with one or more core linkers 12,14 that are configured to form a linkage with a capture molecule 2 and/or an anchor molecule 18.

In some embodiments, one or more core linkers 12, 14 are linked to one or more capture molecules through a chemical bond. In some embodiments, at least one of the one or more core linkers 12, 14 comprises a core reactive molecule. In some embodiments, each core reactive molecule independently comprises an amine, a thiol, a DBCO, a NHS ester, a maleimide, biotin, an azide, an acrydite, a single stranded nucleic acid (e.g., RNA or DNA) of specific sequence, or a polymer (e.g., polyethylene glycol (PEG) or one or more polymerization initiators). In some embodiments, at least one of the one or more core linkers comprises a DNA sequence domain.

With reference to FIG. 1A, in some embodiments, the core structure 13 is linked to 1) a capture barcode 20 at a prescribed first location on the core structure, and optionally 2) an anchor molecule 18 at a prescribed second location on the core structure. In some embodiments, a specified first core linker 12 is disposed at the first location on the core structure. In some embodiments, one or more core molecules at the first location are modified to form a linkage with the first core linker 12. In some embodiments, the first core linker 12 is an extension of the core structure 13.

In some embodiments, a specified third core linker 14 is disposed at the second location on the core structure 13. In some embodiments, one or more core molecules at the second location is modified to form a linkage with the third core linker 14. In some embodiments, the third core linker 12 is an extension of the core structure 13. In some embodiments, the first location is disposed on a first side of the core structure 13, and the optional second location is disposed on a second side of the core structure 13.

With reference to FIG. 1B, in some embodiments, the capture molecule 2 comprises a protein, a peptide, an antibody, an aptamers (RNA and/or DNA), a fluorophore, a nanobody, a darpin, a catalyst, a polymerization initiator, a polymer like PEG, an organic molecule, or combinations thereof. In some embodiments, the capture molecule comprises a modified aptamer. In some embodiments, the capture molecule comprises a SOMAmer^{®}. In some embodiments, the one or more capture molecules comprises a combination of aptamers and modified aptamers, including a combination of SOMAmers^{®} and non-SOMAmer^{®} aptamers. In some embodiments, the modified aptamers comprise a class of nucleic acid-based protein binding reagents which are chemically modified to provide a unique fingerprint as an affinity binder. In some embodiments, modified aptamer assays transform protein concentrations in a mixture into a DNA signature which can then be quantified, for example by using commercially available DNA microarray platforms. In some embodiments, the modified aptamers comprise a dual nature: a) specifically shaped protein-binding folded entities with chemically modified properties, and b) unique nucleic acid sequences which are designed to be recognized by hybridization probes. In some embodiments, the dual nature of the modified aptamers make them a powerful tool for highly multiplexed (>1000 plexity) protein quantification. In some embodiments, the capture molecule comprises a unique shape and chemical properties configured to recognize and bind with a particular analyte molecule (e.g., protein). In some embodiments, the binding between the capture molecule and analyte molecule forms a capture molecule - analyte molecule complex.

In some embodiments, the anchor molecule comprises a reactive molecule. In some embodiments, the anchor molecule 18 comprises a reactive molecule. In some embodiments, the anchor molecule 18 comprises a DNA strand comprising a reactive molecule. In some embodiments, the anchor molecule 18 comprises an amine, a thiol, a DBCO, a NHS ester, a maleimide, biotin, an azide, an acrydite, a single stranded nucleic acid (e.g., RNA or DNA) of specific sequence, or a polymer (e.g., polyethylene glycol (PEG) or one or more polymerization initiators). In some embodiments, the anchor molecule 18 comprises a protein, a peptide, an antibody, an aptamers (RNA and DNA), a flourophore, a nanobody, a darpin, a catalyst, a polymerization initiator, a polymer like PEG, an organic molecule or combinations thereof.

In some embodiments, each component of the supramolecular structure may be independently modified or tuned. In some embodiments, modifying one or more of the components of the supramolecular structure may modify the 2D and 3D geometry of the supramolecular DNA origami structure itself. In some embodiments, modifying one or more of the components of the supramolecular structure may modify the 2D and 3D geometry of the core structure. In some embodiments, such capability for independently modifying the components of the supramolecular structure enables precise control over the organization of one or more supramolecular structures on solid surfaces (e.g., planar surfaces or microparticles) and 3D volumes (e.g., within a hydrogel matrix).

### Capture barcode

As shown in FIGS. 1A-B, in the present invention, the capture molecule 2 is linked to the core structure 13 through a capture barcode 20. In some embodiments, the capture barcode 20 forms a linkage with the capture molecule 2, and the capture barcode 20 forms a linkage with the core structure 13. In some embodiments, the capture barcode 20 is configured to form a linkage with a particular capture molecule (e.g., aptamer). In some embodiments, the capture barcode is configured to form a linkage with a particular capture molecule through a chemical linkage. In some embodiments, the chemical linkage comprises maleiamide-thiol, DBCO-Azide, Amine-NHS ester. In some embodiments, the capture barcode is configured to hybridize with a capture molecule. In some embodiments, the capture barcode further provides a barcode for the supramolecular molecular structure, and can be used to map the location of said supramolecular structure, for example when a plurality of supramolecular structures are placed on multiple binding locations on a planar substrate.

In some embodiments, the capture barcode 20 comprises a first capture linker 11, a second capture linker 6, and a capture bridge 7. In some embodiments, the first capture linker 11 comprises a reactive molecule. In some embodiments, the first capture linker 11 comprises a reactive molecule comprising an amine, a thiol, a DBCO, a NHS ester, a maleimide, an azide, an acrydite, a single stranded nucleic acid (e.g., RNA or DNA) of specific sequence, or a polymer (e.g., polyethylene glycol (PEG) or one or more polymerization initiators). In some embodiments, the first capture linker 11 comprises a DNA sequence domain. In some embodiments, the second capture linker 6 comprises a reactive molecule. In some embodiments, the second capture linker 6 comprises a reactive molecule comprising an amine, a thiol, a DBCO, a NHS ester, biotin, a maleimide, an azide, an acrydite, a single stranded nucleic acid (e.g., RNA or DNA) of specific sequence, or a polymer (e.g., polyethylene glycol (PEG) or one or more polymerization initiators). In some embodiments, the second capture linker 6 comprises a DNA sequence domain. In some embodiments, the capture bridge 7 comprises a polymer. In some embodiments, the capture bridge 7 comprises a unique barcode sequence that can be used to map the location of a supramolecular structure, and/or that is configured to form a linkage with a particular capture molecule. In some embodiments, the capture bridge 7 comprises a polymer that comprises a nucleic acid (e.g., DNA or RNA) of a specific sequence. In some embodiments, the capture bridge 7 comprises a polymer such as PEG. In some embodiments, the first capture linker 11 is attached to the capture bridge 7 at a first terminal end thereof, and the second capture linker 6 is attached to the capture bridge 7 at a second terminal end thereof. In some embodiments, the first capture linker 11 is attached to the capture bridge 7 via a chemical bond. In some embodiments, the second capture linker 6 is attached to the capture bridge 7 via a chemical bond. In some embodiments, the first capture linker 11 is attached to the capture bridge 7 via a physical attachment. In some embodiments, the second capture linker 6 is attached to the capture bridge 7 via a physical attachment.

In some embodiments, the capture barcode 20 is linked to the core structure 13 through a linkage between the first capture linker 11 and the first core linker 12. In some embodiments, as described herein, the first core linker 12 is disposed at a first location on the core structure 13. In some embodiments, the first capture linker 11 and first core linker 12 are linked together through a chemical bond. In some embodiments, the first capture linker 11 and first core linker 12 are linked together through a covalent bond. In some embodiments, the linkage between the first capture linker 11 and first core linker 12 is reversible upon being subjected to a trigger. In some embodiments, the trigger comprises interaction with a deconstructor molecule ("capture deconstructor molecule") or exposure to a trigger signal. In some embodiments, the capture deconstructor molecule comprises a nucleic acid (DNA or RNA), a peptide, a small organic molecule, or combinations thereof. In some embodiments the trigger signal comprises an optical signal. In some embodiments, the trigger signal comprises an electrical signal, microwave signal, ultraviolet illumination, visible illumination or near infra-red illumination.

In some embodiments, the capture barcode 20 is linked to the capture molecule 2 through a linkage between the second capture linker 6 and a third capture linker 5 that is bound to the capture molecule 2. In some embodiments, the third capture linker 5 comprises a reactive molecule. In some embodiments, the third capture linker 5 comprises a reactive molecule comprising an amine, a thiol, a DBCO, a NHS ester, a maleimide, biotin, an azide, an acrydite, a single stranded nucleic acid (e.g., RNA or DNA) of specific sequence, or a polymer (e.g., polyethylene glycol (PEG) or one or more polymerization initiators). In some embodiments, the third capture linker 5 comprises a DNA sequence domain. In some embodiments, the capture molecule 2 is bound to the third capture linker 5 through a chemical bond. In some embodiments, the capture molecule 2 is bound to the third capture linker 5 through a covalent bond. In some embodiments, the second capture linker 6 and third capture linker 5 are linked together through a chemical bond. In some embodiments, the second linker 6 and third capture linker 5 are linked together through a covalent bond. In some embodiments, the linkage between the second capture linker 6 and third capture linker 5 is reversible upon being subjected to a trigger. In some embodiments, the trigger comprises interaction with a deconstructor molecule ("capture barcode release molecule" or exposure to a trigger signal. In some embodiments, the capture barcode release molecule comprises a nucleic acid (DNA or RNA), a peptide, a small organic molecule, or combinations thereof. In some embodiments the trigger signal comprises an optical signal. In some embodiments, the trigger signal comprises an electrical signal, microwave signal, ultraviolet illumination, visible illumination or near infra-red illumination.

In some embodiments, the capture barcode 20 is hybridized to the capture molecule 2, such as nucleic acid hybridization. In some embodiments, the capture barcode 20 is linked to the capture molecule 2 via hybridization, such as nucleic acid hybridization. In some embodiment, the capture barcode 20 is linked to the capture molecule 2 via covalent linkage between the molecule 5 and 6, both of which could be pair of molecules that specifically react with each other, for example DBCO-Azide, Amine-NHS Ester, Thiol-Maleimide.

In some embodiments, being subject to a trigger breaks the linkage between the first capture linker 11 and first core linker only 12, thereby breaking the capture molecule linkage with the core nanostructure 13 at the first location. In some embodiments, the capture barcode 20, when separated from the core structure 13 and the capture molecule 2, is configured to provide a signal for detecting an analyte molecule. In some embodiments, the signal as provided from the capture barcode 20 is a DNA signal.

### Anchor Barcode

As shown in FIG. 1, in some embodiments, the anchor molecule 18 is linked to the core structure 13 through an anchor barcode. In some embodiments, the anchor barcode forms a linkage with the anchor molecule 18, and the anchor barcode forms a linkage with the core structure 13. In some embodiments, the anchor barcode provides a barcode for the supramolecular molecular structure, and can be used to map the location of said supramolecular structure, for example when a plurality of supramolecular structures are placed on multiple binding locations on a planar substrate.

In some embodiments, the anchor barcode comprises a first anchor linker 15, a second anchor linker 17, and an anchor bridge 16. In some embodiments, the first anchor linker 15 comprises a reactive molecule. In some embodiments, the first anchor linker 15 comprises a reactive molecule comprising an amine, a thiol, a DBCO, a NHS ester, a maleimide, biotin, an azide, an acrydite, a single stranded nucleic acid (e.g., RNA or DNA) of specific sequence, or a polymer (e.g., polyethylene glycol (PEG) or one or more polymerization initiators). In some embodiments, the first anchor linker 15 comprises a DNA sequence domain. In some embodiments, the second anchor linker 17 comprises a reactive molecule. In some embodiments, the second anchor linker 17 comprises a reactive molecule comprising an amine, a thiol, a DBCO, a NHS ester, a maleimide, biotin, an azide, an acrydite, a single stranded nucleic acid (e.g., RNA or DNA) of specific sequence, or a polymer (e.g., polyethylene glycol (PEG) or one or more polymerization initiators). In some embodiments, the second anchor linker 17 comprises a DNA sequence domain. In some embodiments, the anchor bridge 16 comprises a polymer. In some embodiments, the anchor bridge 16 comprises a polymer that comprises a nucleic acid (DNA or RNA) of a specific sequence. In some embodiments, the anchor bridge 16 comprises a polymer such as PEG. In some embodiments, the first anchor linker 15 is attached to the anchor bridge 16 at a first terminal end thereof, and the second anchor linker 17 is attached to the anchor bridge 16 at a second terminal end thereof. In some embodiments, the first anchor linker 15 is attached to the anchor bridge 16 via a chemical bond. In some embodiments, the second anchor linker 17 is attached to the anchor bridge 16 via a physical attachment. In some embodiments, the first anchor linker 15 is attached to the anchor bridge 16 via a chemical bond. In some embodiments, the second anchor linker 17 is attached to the anchor bridge 16 via a physical attachment.

In some embodiments, the anchor barcode is linked to the core structure 13 through a linkage between the first anchor linker 15 and the third core linker 14. In some embodiments, as described herein, the third core linker 14 is disposed at a third location on the core structure 13. In some embodiments, the first anchor linker 15 and third core linker 14 are linked together through a chemical bond. In some embodiments, the first anchor linker 15 and third core linker 14 are linked together through a covalent bond. In some embodiments, the linkage between the first anchor linker 15 and third core linker 14 is reversible upon being subjected to a trigger. In some embodiments, the trigger comprises interaction with a deconstructor molecule ("anchor deconstructor molecule" or exposure to a trigger signal. In some embodiments, the anchor deconstructor molecule comprises a nucleic acid (DNA or RNA), a peptide, a small organic molecule, or combinations thereof. In some embodiments the trigger signal comprises an optical signal. In some embodiments, the trigger signal comprises an electrical signal, microwave signal, ultraviolet illumination, visible illumination or near infra-red illumination.

In some embodiments, the anchor barcode is linked to the anchor molecule 18 through a linkage between the second anchor linker 17 and the anchor molecule 18. As disclosed herein, in some embodiments, the anchor molecule comprises a reactive molecule, a reactive molecule, a DNA sequence domain, a DNA sequence domain comprising a reactive molecule, or combinations thereof. In some embodiments, the anchor molecule 18 is bound to the second anchor linker 17 through a chemical bond. In some embodiments, the anchor molecule 18 is bound to the second anchor linker 17 through a covalent bond. In some embodiments, the linkage between the second anchor linker 17 and anchor molecule 18 is reversible upon being subjected to a trigger. In some embodiments, the trigger comprises interaction with a deconstructor molecule ("anchor barcode release molecule" or exposure to a trigger signal. In some embodiments, the anchor barcode release molecule comprises a nucleic acid (DNA or RNA), a peptide, a small organic molecule, or combinations thereof. In some embodiments the trigger signal comprises an optical signal. In some embodiments, the trigger signal comprises an electrical signal, microwave signal, ultraviolet illumination, visible illumination or near infra-red illumination.

In some embodiments, being subject to a trigger breaks the linkage between the first anchor linker 15 and third core linker 14 only, thereby breaking the anchor molecule linkage with the core structure 13 at the third location.

In some embodiments, the capture deconstructor molecule and capture barcode release molecule comprise the same type of molecule. In some embodiments, the capture deconstructor molecule and capture barcode release molecule comprise different types of molecules. In some embodiments, the capture deconstructor molecule, capture barcode release molecule, anchor deconstructor molecule, and anchor barcode release molecule comprise the same type of molecules. In some embodiments, the capture deconstructor molecule, capture barcode release molecule, anchor deconstructor molecule, and anchor barcode release molecule comprise different types of molecules. In some embodiments, any combination of the capture deconstructor molecule, capture barcode release molecule, anchor deconstructor molecule, and anchor barcode release molecule comprise the same type of molecules.

In some embodiments, the core structure comprises a scaffolded DNA origami, wherein a circular ssDNA molecule, called "scaffold" strand, is folded into a predefined 2D or 3D shape by interacting with 2 or more short ssDNA, called "staple" strands, which interact with specific sub-sections of the ssDNA "scaffold" strand.

In some embodiments of a supramolecular DNA origami structure, the core structure comprises a DNA origami. In some embodiments, the core structure 13 comprises a first core linker 12 comprising a DNA sequence domain. In some embodiments, the first core linker 12 is complementary to a first capture linker 11 on the capture barcode strand 20. In some embodiments, the capture barcode strand 20 comprises a DNA strand comprising the first capture linker 11 and a second capture linker at either end of said capture barcode strand. In some embodiments, the first capture linker 11 comprises a DNA sequence domain. In some embodiments, the second capture linker 6 comprises a DNA sequence domain. In some embodiments, the capture barcode strand 20 further comprises a unique capture barcode sequence 7 in between the first and second capture linkers 11, 6. In some embodiments, the unique capture barcode sequence 7 comprises a nucleic acid (DNA or RNA) of a specific sequence. In some embodiments, the unique capture barcode sequence 7 comprises a polymer such as PEG. In some embodiments, the capture barcode 20 comprises a short domain called the toehold ("TH"). In some embodiments, the capture barcode sequence 7 comprises the toehold ("TH").

In some embodiments, the second capture linker 6 is complementary to a third capture linker 5. In some embodiments, the third capture linker 5 is a DNA sequence domain. In some embodiments, a capture molecule 2 is bound to the third capture linker 5. In some embodiments, the capture molecule 2 is covalently bound to the third capture linker 5. In some embodiments, the capture molecule 2 is bound to the capture barcode 20 directly. In some embodiments, the capture molecule 2 is bound to the capture barcode sequence 7 directly.

In some embodiments, the core structure comprises a second core linker 14 that comprises a DNA sequence domain. In some embodiments, the second core linker 14 is complementary to a first anchor linker 15 on the anchor barcode strand 22. In some embodiments, the anchor barcode strand 22 comprises a DNA strand comprising the first anchor linker 15 and a second anchor linker 17 at either end of the anchor barcode section 22. In some embodiments, the first anchor linker 15 comprises a DNA sequence domain. In some embodiments, the second anchor linker 17 comprises a DNA sequence domain. In some embodiments, the anchor barcode strand 22 further comprises a unique anchor barcode sequence 16 in between the first and second anchor linkers 15, 17. In some embodiments, the anchor barcode 22 comprises a short domain called the toeholds ("TH"). In some embodiments, the anchor barcode sequence 16 comprises the toeholds ("TH"). In some embodiments, the unique detector barcode sequence 16 comprises a nucleic acid (DNA or RNA) of a specific sequence. In some embodiments, the unique detector barcode sequence 16 comprises a polymer such as PEG.

In some embodiments, the second anchor linker 17 is complementary to the anchor molecule 18. In some embodiments, the anchor molecule 18 comprises a DNA sequence domain. In some embodiments, the anchor molecule 18 is linked to a terminal modification. In some embodiments, the terminal modification comprises a reactive molecule. In some embodiments, the terminal modification comprises a reactive molecule comprising an amine, a thiol, a DBCO, a NHS ester, a maleimide, biotin, an azide, an acrydite, a single stranded nucleic acid (e.g., RNA or DNA) of specific sequence, or a polymer (e.g., polyethylene glycol (PEG) or one or more polymerization initiators).

### Methods for Detecting Analyte Molecules

As described herein, in some embodiments, one or more supramolecular structures enable the detection of one or more analyte molecules in a sample. In some embodiments, the supramolecular structures each comprise a supramolecular DNA origami structure. In some embodiments, the supramolecular structures move from a ground state to an excited state via linkage with a given analyte molecule (via a corresponding capture molecule that is linked to said supramolecular structure). In some embodiments, the supramolecular structures in an excited state are configured to convert information about the presence of said given analyte molecule in a sample to a signal. In some embodiments, the signal comprises a fluorescent label-based signal, a label-free signal, or combination thereof. In some embodiments, the identification and/or quantification of a given analyte molecule in a sample using a signal corresponds to a capture barcode located on a supramolecular DNA origami structure, wherein the location of a plurality of supramolecular structure are mapped according to the respective capture barcode. In some embodiments, each capture barcode is configured to form a linkage with a particular capture molecule. In some embodiments, the capture molecule comprises a modified aptamer.

In some embodiments, detecting the presence of an analyte molecule or a plurality of analyte molecules, as described herein, comprises optical and/or electronic readout of signals from multiple fluorescent labeling and/or label-free events that correspond to one or more analyte molecules linked with a corresponding supramolecular structure. In some embodiments, the one or more analyte molecules, linked with a corresponding supramolecular structure, are immobilized on solid support(s) or planar solid substrate(s), whereon the corresponding supramolecular structures and capture molecules are immobilized in a predetermined fashion. As used herein, the term "capture molecule" and "recognition molecule" are used interchangeably.

In some embodiments, a plurality of analyte molecules are simultaneously detected in a sample through multiplexing, wherein a plurality of supramolecular structures enable a plurality of signals (e.g., optical or electrical) to be detected for analyte molecule identification. In some embodiments, methods described herein for detecting analytes in a sample provide a high-throughput and high-multiplexing capability by using a plurality of supramolecular structures (e.g., supramolecular DNA origami structures). In some embodiments, the high-throughput and high-multiplexing capability provides high accuracy for analyte molecule detection and quantification. In some embodiments, methods described herein for detecting analytes in a sample are configured to characterize and/or identify biopolymers, including proteins molecules, quickly and at high sensitivity and reproducibility. In some embodiments, the plurality of supramolecular DNA origami structures are configured to limit cross-reactivity associated errors. In some embodiments, such cross-reactivity associated errors comprise capture molecules of a supramolecular DNA origami structure interacting with capture molecules of another supramolecular DNA origami structure (e.g., intermolecular interactions). In some embodiments, each core structure of the plurality of supramolecular DNA origami structures is identical to one another. In some embodiments, the structural, chemical, and physical property of each supramolecular DNA origami structure is explicitly designed. In some embodiments, identical core structures have a prescribed shape, size, molecular weight, prescribed number of capture molecules, predetermined distance between corresponding capture molecules (as described herein), or combinations thereof, so as to limit the cross-reactivity between supramolecular DNA origami structures. In some embodiments, the molecular weight of every core structure is identical and precise up to the purity of the core molecules. In some embodiments, each core structure has at least one capture molecule.

In some embodiments, the plurality of supramolecular DNA origami structures are each configured to form a linkage with different analyte molecules from each other (via the corresponding capture molecule). In some embodiments, the state change (from unexcited to excited) is driven primarily by the linkage between a capture molecule (linked with the supramolecular structure) and a particular analyte molecule. In some embodiments, the plurality of supramolecular structures might share structural similarities due to certain subcomponents being the same, however the linkage between an analyte molecule from the sample and supramolecular structure is defined by the corresponding capture molecule. In some embodiments, as described herein, each capture barcode on a supramolecular structure is configured to form a linkage with the same particular capture molecule. In some embodiments, each capture molecule on a given supramolecular DNA origami structure may specifically interact with a particular analyte molecule in the sample, leading to a state change of supramolecular structure upon interacting with the particular analyte molecule. In some embodiments, each supramolecular structure comprises unique DNA barcodes (e.g., capture barcode) corresponding to the respective capture molecule. In some embodiments, a capture molecule on a given supramolecular DNA origami structure is designed to interact with only one type of analyte molecule in the sample In some embodiments, a capture molecule on a given supramolecular DNA origami structure is designed to interact with more than one type of analyte molecule in the sample.

In some embodiments, each supramolecular DNA origami structure is configured for single-molecule sensitivity to ensure the highest possible dynamic range needed to quantitatively capture the wide range of molecular concentrations within a typical complex biological sample. In some embodiments, single-molecule sensitivity comprises a given supramolecular DNA origami structure configured to shift from a ground state to an excited state through interaction between a corresponding capture molecule (that is linked to the given supramolecular structure) and a single analyte molecule, as described herein . In some embodiments, the plurality of supramolecular DNA origami structures limit or eliminate the manipulation of the sample needed to reduce non-specific interaction as well as any user induced errors.

In some embodiments, the plurality of supramolecular structures are provided in a solution. In some embodiments, the plurality of supramolecular structures are attached to one or more substrates. In some embodiments, the plurality of supramolecular structures are attached to one or more widgets. In some embodiments, the plurality of supramolecular structures are attached to one or more solid substrates, one or more polymer matrices, one or more molecular condensates, or combinations thereof. In some embodiments, the one or more polymer matrices comprises one or more hydrogel particles. In some embodiments, the one or more polymer matrices comprises one or more hydrogel beads. In some embodiments, the one or more solid substrates comprises one or more planar substrates. In some embodiments, the one or more solid substrates comprises one or more microbeads. In some embodiments, the one or more solid substrates comprises one or more microparticles.

In some embodiments, the sample and supramolecular DNA origami structures are incubated in an incubator with prescribed environmental conditions. In some embodiments, the sample is incubated with the supramolecular DNA origami structures for a time period from about 30 seconds to about 24 hours. In some embodiments, the sample is incubated with the supramolecular DNA origami structures for a time period from about 30 seconds to about 1 minute, from about 1 minute to about 5 minutes, from about 5 minutes to about 30 minutes, from about 30 minutes to about 1 hr, from about 1hr to about 5 hours, from about 5 hours to about 12 hours, from about 12 hours to about 24 hours, from about 24 hours to about 48 hours.

In some embodiments, the method for detecting analyte molecules comprises cleaving the capture barcode from a corresponding capture molecule that has interacted with an analyte molecule. In some embodiments, the capture barcodes are cleaved from the corresponding capture molecules through nucleic acid (DNA/RNA) strand displacement, optical cleavage, chemical cleavage, or a combination thereof.

In some embodiments, the cleaved capture barcodes are isolated from a solution comprising the supramolecular DNA origami structures. In some embodiments, the cleaved capture barcodes are isolated from the solution through polyethylene glycol (PEG) precipitation. In some embodiments, the cleaved capture barcodes provide a signal that correlates to the respective analyte molecule bound to the respective capture molecule. In some embodiments, as described herein, the capture barcode comprises a DNA strand. In some embodiments, the capture barcode provides a DNA signal correlating to the analyte molecule. In some embodiments, the isolated capture barcodes are analyzed to identify and/or quantify the corresponding analyte molecules in the sample. In some embodiments the analysis of the isolated capture barcodes comprises genotyping, qPCR, sequencing, or combinations thereof.

In some embodiments, arraying the capture molecules (e.g., modified aptamers) on a DNA origami placement based array through DNA hybridization or other attachment techniques (as described herein) provides an alternate platform to using DNA microarray techniques for quantification of protein binding events, and resultant DNA signature embedded within modified aptamers. In some embodiments, a solution-based assay could then be transformed into a chip-based assay as an alternate to using bead pull-downs and UV photo cleaving strategies.

### Detection of Analyte Molecules using a Surface Assay

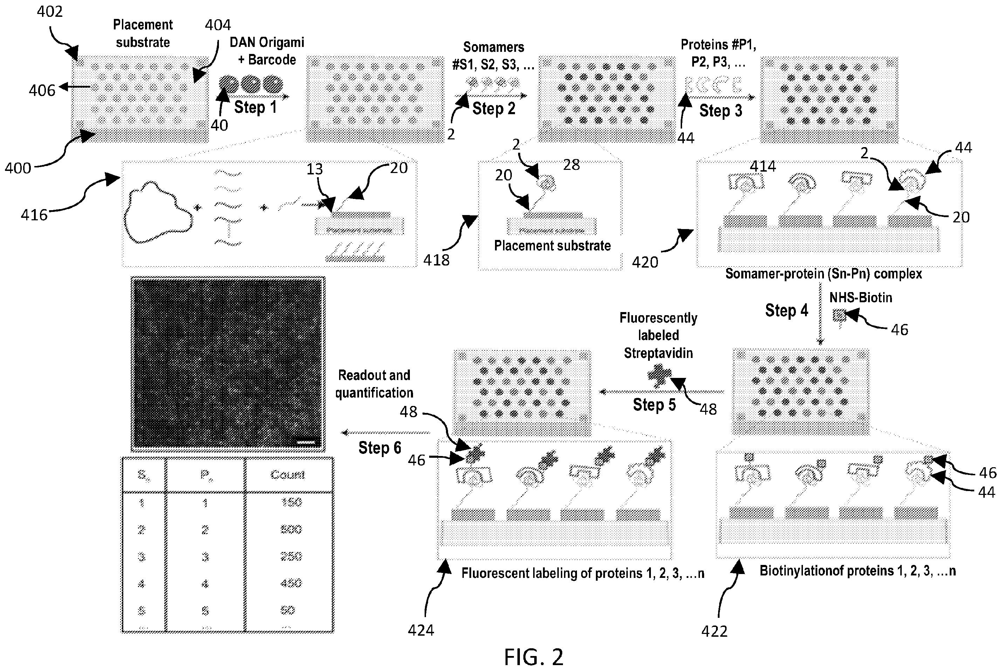

FIG. 2 provides an exemplary illustration of a method for detecting analyte molecules in a sample using a surface based assay that uses supramolecular structures, as described herein, for single-molecule counting of analytes in the sample (i.e. detecting analyte molecules in the sample at a single molecule resolution). In some embodiments, the supramolecular structures comprise a core structure 13 comprising a DNA origami core. In some embodiments, a planar substrate 400 is provided comprising (a) fiduciary markers 402 that serves as a reference coordinates for all the features on the substrate; (b) a defined set of micropatterned binding sites 406 where individual core structures (e.g., DNA origami) may be immobilized; and/or (c) background passivation 404 that minimizes or prevents interaction between the surface of the substrate 400 and the supramolecular structure (e.g., capture molecules, core structure molecules). In some embodiments, the fiduciary markers 402 comprise geometric features defined on a surface to be used as reference features for other features on the substrate 400. In some embodiments, the fiduciary markers 402 are coated with a polymer or self-assembled monolayer that does not interact with a core structure or other molecules of the supramolecular structure (e.g., DNA origami). In some embodiments, the background passivation 404 minimizes or prevents interaction between the surface of the substrate and analyte molecules of the sample. In some embodiments, in addition to background passivation required for preferential supramolecular structure binding (e.g., preferential DNA origami binding) to the binding sites 406 on the substrate 400, the substrate 400 is chemically treated with various blocking reagents to promote specific interactions of capture molecules (e.g., aptamers), analyte molecules (e.g., protein analytes), and labeling entities (e.g., NHS-biotin and streptavidin) with the supramolecular structure (e.g., DNA origami) molecules and/or molecules linked thereto. In some embodiments, the planar substrate 400 comprises differential chemistry in the binding sites 406. In some embodiments, the planar substrate 400 is fabricated through lithography processes as known in the art. In some embodiments, the planar substrate comprises optical or electrical devices like FET, ring resonators, photonic crystals or microelectrode, which may be placed on the substrate prior to the formation of the binding sites 406. In some embodiments, the binding sites 406 are micropatterned on the planar substrate 400. In some embodiments, the binding sites 406 on the surface are in a periodic pattern. In some embodiments, the binding sites on the surface are in a non-periodic pattern (e.g., random). In some embodiments, a minimum distance is specified between any two binding sites. In some embodiments, the minimum distance between any two binding sites is at least about 200 nm. In some embodiments, the minimum distance between any two binding sites is from at least about 40 nm to about 5000 nm. In some embodiments, the geometric shape of the binding sites comprises a circle, square, triangle or other 2-D or 3-D polygon shapes. In some embodiments, the chemical groups that are used for passivation comprise neutrally charged molecules like a Tri-methyl silyl (TMS), an uncharged polymer like PEG a zwitterionic polymer like, or combinations thereof. In some embodiments, the chemical group used to define the binding site comprises a silanol group, carboxyl group, thiol, other groups, or combinations thereof.

In some embodiments, a single supramolecular structure 40 is attached to a respective binding site 406 (Step 1). Accordingly, in some embodiments, a plurality of supramolecular structures 40 are each attached to a corresponding binding site 406 on the substrate 400. Reference character 416 provides a depiction of the components of the supramolecular structure 40, individually and as assembled and arranged on the planar substrate. In some embodiments, the supramolecular structure comprises the components and arrangement as described in FIGS. 1A-B herein. In some embodiments, the supramolecular structure 40 comprises a core structure comprising a DNA origami (e.g., M13mp18 scaffold and staples), wherein the supramolecular structures are attached onto each of the binding sites 406 using DNA origami placement technique (step 1). In some embodiments, the supramolecular structure 40 is assembled prior to being attached to a respective binding site 406. In some embodiments, the DNA origami comprises a unique shape and dimension, so as to facilitate binding to a binding site using the DNA origami placement technique. In some embodiments, DNA origami placement comprises a directed self-assembly technique for organizing individual DNA origami (e.g., a core structure) on a surface (e.g., micropatterned surface). In some embodiments, alternatively to the DNA origami placement, a reactive group of the supramolecular structure 40 is bound to a DNA origami that has been pre-organized on a binding site 406. In some embodiments, the reactive group comprises the anchor molecule as described herein (e.g., FIG. 1). In some embodiments, both of these methods for binding a supramolecular structure 40 to a corresponding binding site 406 rely on the ability to organize one or more molecules on a micropatterned binding site using the DNA origami placement technique. In some embodiments, the planar substrate could be stored for a significant period after this step, in a clean environment.

In some embodiments, the supramolecular structures 40 are placed onto the binding sites 406 with high efficiency of single molecule binding in said binding sites 406.

With reference to reference character 416, in some embodiments, the supramolecular structures comprise a single or a plurality of capture barcodes. In some embodiments, all the capture barcodes on a given supramolecular structure is configured to form a linkage with the same type of capture molecule, such that all the capture barcodes on a given supramolecular structure are configured to form a linkage with the same type of analyte molecule (via the specific type of capture molecule). In some embodiments, the supramolecular structure comprises one or more capture barcodes, and further comprises one or more additional barcode strands. In some embodiments, the supramolecular structures comprise one or more anchor barcodes. In some embodiments, the supramolecular structures on a substrate are mapped via the capture barcodes, anchor barcode, and/or other barcodes linked with supramolecular structures, so as to catalog the position of each specific analyte binding position on the substrate 400 (e.g., micro patterned surface). Accordingly, a map of the binding location(s) 406 for a specific capture molecule, and thus specific analyte molecule, on the substrate 400, is created via a unique capture barcode and/or another barcode (e.g., anchor barcode, additional barcode) linked with the supramolecular structure 40. In some embodiments, a dye-based hybridization assay or sequencing of the barcode region is used to create a map of the spatial locations corresponding to unique capture molecule binding locations 406 on the substrate 400. In some embodiments, said mapping of the capture molecule binding locations is done at the site of manufacture of the substrate 400 or prior to performing the assay. In some embodiments, each substrate can have a unique ID which can be looked up for mapping information. Alternately, mapping can be performed after the capture molecule has been immobilized on the substrate 400. In some embodiments, the supramolecular structures 40 each comprise a single or a plurality of capture sites for a specific capture molecule, as described herein. In some embodiments, one or more supramolecular structures 40 comprise a capture site for a specific capture molecule.

In some embodiments, capture molecules 2(as described herein) are contacted with the planar substrate 400 (step 2). In some embodiments, as described herein, the capture molecules 2 comprise aptamers, including modified aptamers, or other affinity binders. In some embodiments, the modified aptamers comprise SOMAmers^{®}. In some embodiments, the capture molecules 2 are contacted with the planar substrate using a flow-cell. In some embodiments, the capture molecules are provided in a solution that is allowed to flow over the substrate 40, and thus, also allowed to flow over the supramolecular structures 40. In some embodiments, the capture molecules are hybridized onto the substrate (40), which in some instances, is similar to a process when contacting capture molecules with a DNA microarray pattern. In some embodiments, the capture molecules are linked with the supramolecular structures through the linkage as described in FIGS. 1A-B herein. As shown in FIG. 2, the different capture molecules are identified as S₁, S₂,...Sₙ. In some embodiments, the capture molecules are incubated on the planar substrate 400 with the supramolecular DNA origami structures 40 attached to the binding sites 416. In some embodiments, the incubation period is from about 30 seconds to about 24 hours. In some embodiments, the incubation period is from about 30 seconds to about 1 minute, from about 1 minute to about 5 minutes, from about 5 minutes to about 30 minutes, from about 30 minutes to about 1 hr, from about 1hr to about 5 hours, from about 5 hours to about 12 hours, from about 12 hours to about 24 hours, from about 24 hours to about 48 hours.

In some embodiments, a capture molecule interacts with a corresponding capture barcode on the plurality of supramolecular DNA origami structures, such that the capture molecule is captured by the capture barcode. In some embodiments, a capture barcode forms a linkage with a corresponding capture molecule, such that the capture molecule is captured by the capture barcode (see reference character 418). Accordingly, in some embodiments, the capture molecule is immobilized on the substrate 400 via the linkage with the supramolecular structure (via the corresponding capture barcode). In some embodiments, the capture molecule is captured by the capture barcode via hybridization. In some embodiments, the capture molecule is captured by the capture barcode via a third capture linker, as described herein in FIGS. 1A-B. In some embodiments, each capture barcode is configured to interact with a particular capture molecule (e.g., aptamer, affinity binder, etc.).

In some embodiments, interferometric scattering microscopy (iSCAT), which is a method of label-free mass photometry, is used to visualize the interaction (e.g., binding process) between the capture barcodes and corresponding capture molecules in a label-free format. In some embodiments, interferometric scattering microscopy (iSCAT), which is a method of label-free mass photometry, is used to visualize the linkage between the capture barcodes and corresponding capture molecules in a label-free format.

With continued reference to FIG. 2, in some embodiments, a sample (as described herein) comprising analyte molecules 44 is contacted with the planar substrate 400 (step 3). In some embodiments, the sample is contacted with the planar substrate 400 using a flow-cell. In some embodiments, the sample is allowed to flow over the substrate 400 comprising the captured capture molecules 2. In some embodiments, the analyte molecules 44 comprise proteins. In some embodiments, the proteins comprise one or more types of proteins. As show in FIG. 2, the different analyte molecules are identified as P₁, P₂,...Pₙ. In some embodiments, the sample is incubated on the planar substrate 400 with the supramolecular structures 40 (as attached to the corresponding binding sites 416), and the corresponding captured molecules 2. In some embodiments, the incubation period is from about 30 seconds to about 24 hours. In some embodiments, the incubation period is from about 30 seconds to about 1 minute, from about 1 minute to about 5 minutes, from about 5 minutes to about 30 minutes, from about 30 minutes to about 1 hr, from about 1hr to about 5 hours, from about 5 hours to about 12 hours, from about 12 hours to about 24 hours, from about 24 hours to about 48 hours.

In some embodiments, the analyte molecules 44 in the sample, interact with corresponding capture molecules 2 located on the supramolecular DNA origami structures 40 on the planar surface 400.. As described herein, in some embodiments, the analyte molecules 44 comprise proteins. In some embodiments, a single copy of a specific analyte molecule 44 binds with a corresponding capture molecule 2 that was captured by a capture barcode 20 (see reference character 420). As described herein, each capture molecule 2 is configured to bind with a particular analyte molecule 44. In some embodiments, the unique shape and chemical properties of a given capture molecule 2 (e.g., modified aptamer) will recognize and bind with a corresponding analyte molecule 44 (e.g., protein), forming an capture molecule-analyte molecule complex (see reference character 420, with reference to Sₙ-Pₙ complex) at a given binding site 416 on the substrate 400. Accordingly, in some embodiments, the analyte molecules are immobilized on the substrate 400 via the interaction with the capture molecules. In some embodiments, a capture molecule will interact with a specific analyte molecule and bind thereto. In some embodiments, a capture molecule will interact with a specific analyte molecule only and bind thereto. In some embodiments, a capture molecule will directly interact with a specific analyte molecule.

In some embodiments, after the supramolecular structures 40 are linked with the analyte molecules 44 (via the corresponding capture molecules 2), as described herein, the supramolecular structures are then contacted with one or more other identifying molecules so as to identify the supramolecular structures that linked with the analyte molecules in the sample, and thereby identify said analyte molecules found within the sample. In some embodiments, the analyte molecules are identified via the mapped location of the supramolecular structures, as described herein. In some embodiments, the analyte molecules are further quantified in the sample based on the amount of analyte molecules identified across the binding sites 416 of the substrate 400.

In some embodiments, the one or more identifying molecules comprise biotin molecules 46. In some embodiments, the substrate 400 is contacted with biotin molecules such that one or more analyte molecules 44 are subject to biotinylation (step 4), see reference character 422. In some embodiments, being subject to biotinylation corresponds to the analyte molecules 44 interacting with the biotin molecules 46. In some embodiments, the analyte molecules form a linkage with the biotin molecules. In some embodiments, a solution comprising one or more biotin molecules is allowed to flow over the substrate 400. In some embodiments, the analyte molecules 44 are subject to amine biotinylation, sulfhydryl biotinylation, carboxyl biotinylation, glycoprotein biotinylation, oligonucleotide biotinylation, non-specific biotinylation, or a combination thereof. In some embodiments, the one or more biotin molecules comprise NHS-biotin molecules or any other types of biotin molecules. In some embodiments, the one or more biotin molecules comprise amine reactive NHS-biotin molecules. In some embodiments, the one or more amine reactive NHS-biotin molecules label amines by forming permanent amide bonds.

In some embodiments, after the analyte molecules 44 have been subject to biotinylation (e.g., step 4), the analyte molecules 44 are then fluorescently labeled (step 5). In some embodiments, the substrate 400 is contacted with one or more fluorescently labeled molecules 48. In some embodiments, a solution comprising one or more fluorescently labeled molecules 48 is allowed to flow over the substrate 400. In some embodiments, the one or more fluorescent labeling molecules comprise fluorescently labeled streptavidin molecules, fluorescently labeled avidin molecules, or other types of chemistries known for labeling analyte molecules (e.g., proteins) with biotin. In some embodiments, the fluorescently labeled molecules interact ( with the biotin molecules that interacted with the analyte molecules (see reference character 424).

In some embodiments, fluorescently labeling the analyte molecules that are bound with biotin molecules provides a fluorescent signal. In some embodiments, the fluorescent signals generated by the fluorescently labeled molecules is readout (step 6 as shown in FIG. 2) using a fluorescent microscope or any other device known in the art to detect fluorescent signals. In some embodiments, the fluorescent signal detected from a specific binding location 406 on the substrate 400 identifies the capture of a particular analyte molecule (e.g., protein), based on the mapped location of the supramolecular structures 40 and corresponding capture molecules (as described herein). In some embodiments, the captured analyte molecules are quantified based on a cumulative count of the fluorescent signals detected at the corresponding binding locations 406 on the substrate 400. For example, if location X1Y1, X3Y3, and X20Y20 on the substrate 400 corresponds to capture molecule S₁ as mapped through the unique capture barcode on the supramolecular structure 40 (e.g., supramolecular DNA origami structure) molecules at those locations, then fluorescent signals from these three locations following the streptavidin labeling step would result in a count of 3 for analyte molecule P₁ (e.g., Protein P₁).

In addition to or alternative to the fluorescently labeling step described above, in some embodiments, after subjecting captured analyte molecules to biotinylation (i.e. after step 4), the substrate 400 is contacted with one or more molecules or nanoparticles that scatter light, to enable label-free imaging of the analyte molecules 44. In some embodiments, a solution comprising one or more molecules or nanoparticles that scatter light is allowed to flow over the substrate 400. In some embodiments, the one or more molecules or nanoparticles that scatter light comprise streptavidin molecules, avidin molecules, or other types of chemistries known for interacting with biotin molecules. In some embodiments, the one or more molecules or nanoparticles that scatter light comprise streptavidin coated nanoparticle, cluster of streptavidin, avidin coated nanoparticle other molecules and nanoparticles that interact with biotin molecule, or a combination thereof. In some embodiments, the molecules or nanoparticles that scatter light are labelled with Qdots and/or metal nanoparticles to enable label-free imaging of the analyte molecules. In some embodiments, interferometric scattering microscopy (iSCAT) or other types of devices known in the art is used to visualize the complexes formed via the binding between the molecules or nanoparticles that scatter light and biotin molecules (e.g., biotin-streptavidin complex) at the locations of the corresponding analyte molecules 44 that are immobilized on the substrate 400 (i.e., analyte molecules immobilized via interaction with a corresponding capture molecule linked to a supramolecular structure), so as to generate a visual signal. In some embodiments, the visual signal comprises an optical signal, an electrical signal, or both. In some embodiments, the optical signal comprises a microwave signal, an ultraviolet illumination, a visible illumination, a near infrared illumination, scattering of light, or combinations thereof. In some embodiments, visual detection of such a complex from a specific location on the substrate identifies the capture of a particular analyte molecule (e.g., protein), based on the mapped location of the supramolecular structures and corresponding capture molecules (as described herein), thereby identifying the analyte molecules 44 (step 6). In some embodiments, the captured analyte molecules are quantified (step 6) based on a cumulative count of the biotin complexes visually detected at the corresponding binding locations 406 on the substrate 400. For example, if location X1Y1, X3Y3, and X20Y20 on the substrate 400 corresponds to capture molecule S₁ as mapped through the unique capture barcode on the supramolecular structure 40 (e.g., supramolecular DNA origami structure) molecules at those locations, then visually detection of the biotin complexes from these three locations would result in a count of 3 for analyte molecule P₁ (e.g., Protein P₁).

In some embodiments, in lieu of contacting the substrate 400 with biotin molecules, after step 3, the substrate 400 is contacted with a solution comprising a second set of capture molecules. In some embodiments, the second set of capture molecules are fluorescently labeled, unlabeled, or comprise a mixture of both. In some embodiments, the second set of capture molecules are configured to interact with a particular analyte molecule (as described herein for capture molecules). In some embodiments, the second set of capture molecules interact with the corresponding analyte molecules immobilized on the substrate 400, thereby enabling another analyte molecule- capture molecule complex to be formed (i.e., thereby forming a "sandwich" configuration with the analyte molecule located between two capture molecules). As such, in some embodiments, the single molecule patterned surface (substrate 400) with the corresponding capture barcode can be used as a sandwich assay with two capture molecules (e.g., modified aptamers) chemically synthesized to recognize the same analyte molecule. In some embodiments, the second set of capture molecules is allowed to incubate with the substrate 400 (as described herein). In some embodiments, the fluorescently labeled capture molecules from the second set of capture molecules fluorescently label the corresponding analyte molecule that is interacted therewith, so as to generate a fluorescent signal. In some embodiments, a fluorescence readout (step 6) is conducted to identify and quantify the analyte molecules detected on the substrate, as described herein. In some embodiments, unlabeled capture molecules from the second set of capture molecules that interact with the corresponding analyte molecules on the substrate 400 generate a visual signal, wherein the substate 400 is optically interrogated using iSCAT or similar device known in the art, so as to identify and quantify (step 6) the analyte molecules detected on the substrate based on said visual signal, as described herein. As described herein, in some embodiments, the visual signal comprises an optical signal, an electrical signal, or both. In some embodiments, the optical signal comprises a microwave signal, an ultraviolet illumination, a visible illumination, a near infrared illumination, scattering of light, or combinations thereof.

In some embodiments, in another alternative step to contacting the substrate with biotin molecules, after step 3, the substrate 400 is contacted with a solution comprising one or more NHS-dye molecules, or other dye molecules known in the art (such as NHS labelled quantum dots). In some embodiments, the NHS-dye molecules (or other types of dye molecules) are configured to interact with the analyte molecules 44, thereby generating a corresponding fluorescent signal, and thereby enabling a fluorescent readout (step 6) of the analyte molecules immobilized on the substrate 400 (as described herein). In some embodiments, the interaction between the NHS-dye molecules (or other types of dye molecules) and the analyte molecules 44 is a specific interaction. In some embodiments, the fluorescence readout is conducted to identify and quantify the analyte molecules detected on the substrate 400, as described herein.

In some embodiments, introduction of the signaling element, for example fluorescence and/or visual as described herein, leads to a surface on the substrate 400 in which every individual analyte molecule capture event (i.e. linkage between the corresponding capture barcode, capture molecule, and analyte molecule, and subsequent biotinylation or other signal generating event as described herein) leads to a signaling element being present at the location of the respective analyte molecule 44 (on the substrate 400). As described herein, in some embodiments, the signaling element is optically active and can be measured using a microscope or integrated optically sensor within the planar substrate 400. In some embodiments, the signaling element is electrically active and may be measured using an integrated electrical sensor. In some embodiments, the signaling element is magnetically active and may be measured using an integrated magnetic sensor. In some embodiments, each signaling element comprises a fluorescent molecule or microbes, a fluorescent polymer, highly charged nanoparticles, or polymer. In some embodiments, each signal event (at the corresponding binding location 406) is associated with the capture of the same type of analyte molecule (a single copy of the same type of analyte molecule), determined by the corresponding capture molecule. Accordingly, in some embodiments, based on the mapped locations 406 of a given capture barcode 20 on a substrate 400, counting the number of such binding locations 406 where a signaling element is present gives the quantification of the analyte molecule in the sample that corresponds to said given capture barcode.

In some embodiments, between any step as described herein (e.g., steps 1-6 as shown in FIG. 2), the substrate 400 is washed to remove unbound and/or unattached contents from a solution that was contacted with the substrate 400.

In some embodiments, the high-density placement of DNA origami molecules (supramolecular DNA origami structures) on the array (i.e. plurality of binding locations 406 on the substrate 400) enables massively parallel assays for quantification of analyte molecules 44 (e.g., proteins) with plexity limited only by the number of unique capture molecules 2 bound to the supramolecular structures (e.g., origami molecules).

In some embodiments, the method for detecting an analyte as described in FIG.2 enables the detection of a single type of analyte molecule. In some embodiments, the method for detecting an analyte as described in FIG. 2 enables detection of a plurality of types of analyte molecules (multiplexed analyte molecule detection). In some embodiments, each supramolecular structure (e.g. supramolecular DNA origami structure) is barcoded to uniquely identify the respective capture molecules associated therewith, thereby enabling the respective analyte molecule captured to be identified. In some embodiments, each supramolecular DNA origami structure is barcoded using the respective capture barcode and/or anchor molecule.

In some embodiments, the single molecule patterned surface with supramolecular structures (e.g., supramolecular DNA origami structures), which may be DNA origami nanostructures, may be used as a massively multiplexed, high-throughput a systemic evolution of ligands by exponential enrichment ("SELEX") platform for discovery of new capture molecules (e.g., aptamers) that recognize an analyte molecule (e.g., protein) already immobilized on a surface using a capture-detector complex. In some embodiments, the capture-detector complex corresponds to the use of a supramolecular structure comprising a capture molecule and a detector molecule.

In some embodiments, the capture molecule refers to a particular capture molecule (e.g., aptamer), as described herein, configured to interact with a particular analyte molecule. In some embodiments, the detector molecule
refers to a particular capture molecule (e.g., aptamer), as described herein, configured to interact with a particular analyte molecule. In some embodiments, the capture molecule and detector molecules refers to the same type of particular capture molecule (e.g., aptamer), as described herein, configured to interact with a particular analyte molecule. In some embodiments, this capture-detector complex may need to be irreversibly bound and the analyte-capture complex may need to be irreversibly bound as well. In some embodiments, capture barcodes are configured to be separate, wherein one or more separated captured barcodes are analyzed using genotyping, qPCR, sequencing, or combinations thereof. In some embodiments, a plurality of analyte molecules in the sample are detected simultaneously through multiplexing via one or more supramolecular DNA origami structures that shifted to an excited state. In some embodiments, the SELEX platform may need cycling (washing and simultaneous flow through) of tens to thousands of affinity binders.

### Methods for detecting an analyte molecule

Provided herein, is a method for detecting an analyte molecule present in a sample, the method comprising: providing a supramolecular DNA origami structure- arranged in an array format in predetermined locations on a surface- comprising: i) a core structure comprising a single or plurality of molecules, ii) a capture molecule linked to the core structure at a first location which includes a barcode for the purpose of mapping the binding of a specific analyte recognition molecule, iii) an anchor molecule linked to the core structure at a second location which may include a barcode for the purpose of mapping the binding of a specific analyte recognition molecule and/or to bind the DNA origami structure covalently or non-covalently to the surface, and iv) detecting the analyte molecule based on a signal provided by the supramolecular DNA origami structure through fluorophore-labeling or non-labeled techniques of optical detection.

Provided herein, is a method for detecting one or more analyte molecules present in a sample, the method comprising: a) providing a plurality of supramolecular DNA origami structures, each comprising: i) a core structure comprising a single or plurality of molecules for binding unique recognition elements, ii) a capture molecule linked to the core structure at a predetermined location, and iii) detecting the analyte molecule based on a signal provided by the supramolecular DNA origami structure through fluorophore-labeling or label-free techniques of optical detection; b) contacting the recognition elements, i.e. SOMAmers or other affinity-binding entities with the supramolecular DNA origami structures for capture at a single or plurality of locations on said structure through nucleic acid hybridization or other chemical linkages; c) mapping the position of each unique recognition element through fluorescence-based hybridization assays or sequencing sample with the plurality of supramolecular DNA origami structures arranged at predetermined locations on a surface; d) contacting the sample with the recognition elements bound to the supramolecular DNA origami structures at predetermined locations on a surface; e) Generating a readable signal in an optical format through various steps of: i. biotinylating the captured analytes from the sample immobilized by recognition elements at the pre-mapped locations on the surface, ii. Labeling the biotinylated locations with fluorescent, streptavidin moieties; f) Quantifying the analyte concentration through registration of the mapped locations with the signals from specific analyte-binding locations on the surface.

Provided herein, is a method for detecting an analyte molecule present in a sample, the method comprising: providing a supramolecular structure, arranged in an array format in predetermined locations on a surface, comprising: i) a core structure comprising a single or plurality of molecules, ii) a capture molecule linked to the core structure at a first location, wherein the link between he capture molecule and core structure comprises a capture barcode configured to map the interaction of the capture molecule on the supramolecular structure, iii) an anchor molecule linked to the core structure at a second location which may include a barcode for the purpose of mapping the interaction of a specific capture molecule with the capture barcode and/or to bind the DNA origami structure covalently or non-covalently to the surface, iv) contacting the sample with the supramolecular structure such that the capture molecule interacts with the analyte molecule, v) generating a signal based on the interaction between the capture molecule and analyte molecule, and vi) detecting the analyte molecule based on a signal provided by the supramolecular DNA origami structure through fluorophore-labeling or non-labeled techniques of optical detection. In some embodiments, the supramolecular structure comprises a supramolecular DNA origami structure. In some embodiments, the capture molecule comprises an aptamer, including a modified aptamer. In some embodiments, the analyte molecule comprises a protein.

Provided herein, is a method for detecting one or more analyte molecules present in a sample, the method comprising: a) providing a plurality of supramolecular structures, each comprising: i) a core structure comprising a single or plurality of molecules, and ii) a capture barcode linked to the core structure at a predetermined location and configured to form a linkage with a particular capture molecule; b) contacting the supramolecular structures with one or more capture molecules, (e.g., aptamers, modified aptamers, including SOMAmers) or other affinity-binding entities at a single or plurality of locations on a given supramolecular structure through nucleic acid hybridization or other chemical linkages; c) mapping the position of each unique capture molecule through fluorescence-based hybridization assays or sequencing sample with the plurality of supramolecular DNA origami structures arranged at predetermined locations on a surface; e) contacting the sample with the capture molecules linked to the supramolecular structures at the predetermined locations on a surface; e) generating a signal through fluorophore-labeling or label-free techniques of optical detection; f) Identifying and Quantifying the analyte molecule concentration through registration of the mapped locations with the signals from specific analyte-binding locations on the surface. In some embodiments, In some embodiments, detection of the analyte molecules comprises detecting the signal in an optical format through various steps of: i. biotinylating the captured analytes from the sample immobilized by recognition elements at the pre-mapped locations on the surface, ii. Labeling the biotinylated locations with fluorescent, streptavidin moieties. In some embodiments, the supramolecular structure comprises a supramolecular DNA origami structure. In some embodiments, the capture molecule comprises an aptamer, including a modified aptamer. In some embodiments, the analyte molecule comprises a protein.

In some embodiments, any method disclosed herein further comprising quantifying the concentration of the analyte molecule in the sample. In some embodiments, any method disclosed herein further comprising identifying the detected analyte molecule. In some embodiments, any method disclosed herein further comprising detecting the analyte molecule based on the signal when the analyte molecule is present in the sample at a count of a single molecule or higher. In some embodiments, for any method disclosed herein, the sample comprises a complex biological sample and the method provides for single-molecule sensitivity thereby increasing dynamic range and enabling quantitative capture of a range of molecular concentrations within the complex biological sample. In some embodiments, for any method disclosed herein, the analyte molecule comprises a protein, a peptide, a peptide fragment, complexes thereof, or any combinations thereof. In some embodiments, for any method disclosed herein, each supramolecular DNA origami structure is a 2D or 3D nanostructure.

In some embodiments, for any method disclosed herein, each core structure is a nanostructure. In some embodiments, for any method disclosed herein, the plurality of core molecules for each core structure are arranged into a pre-defined shape and/or have a prescribed molecular weight. In some embodiments, the pre-defined shape is configured to limit or prevent cross-reactivity with another supramolecular DNA origami structure. In some embodiments, for any method disclosed herein, the plurality of molecules for each core structure comprises one or more nucleic acid strands, one or more branched nucleic acids, one or more peptides, one or more small molecules, or combinations thereof. In some embodiments, for any method disclosed herein, each core structure independently comprises a scaffolded deoxyribonucleic acid (DNA) origami, a scaffolded ribonucleic acid (RNA) origami, a scaffolded hybrid DNA:RNA origami, a single-stranded DNA tile structure, a multi-stranded DNA tile structure, a single-stranded RNA origami, a multi-stranded RNA tile structure, hierarchically composed DNA or RNA origami with multiple scaffolds, a peptide structure, or combinations thereof.

In some embodiments, the trigger/readout signal comprises an optical signal, an electrical signal, or both. In some embodiments, the trigger optical signal comprises a microwave signal, an ultraviolet illumination, a visible illumination, a near infrared illumination, scattering of light, or combinations thereof.

In some embodiments, for any method disclosed herein, the respective analyte molecule is 1) bound to the capture molecule of the respective supramolecular DNA origami structure through a chemical bond. In some embodiments, for any method disclosed herein, the capture molecule for each supramolecular DNA origami structure comprises a protein, a peptide, an antibody, an aptamer (RNA and/or DNA), a fluorophore, a darpin, a catalyst, a polymerization initiator, a polymer like PEG, or combinations thereof. In some embodiments, the aptamer comprises a modified aptamer. In some embodiments, for any method disclosed herein, wherein for each supramolecular DNA origami structure: a) the capture molecule is linked to the core structure through a capture barcode, wherein the capture barcode comprises a first capture linker, a second capture linker, and a capture bridge disposed between the first and second capture linkers, wherein the first capture linker is bound to a first core linker that is bound to the first location on the core structure, wherein the capture molecule and the second capture linker are linked together through binding to a third capture linker. In some embodiments, the polymer core of the capture bridge independently comprises a nucleic acid (DNA or RNA) of specific sequence or a polymer like PEG. In some embodiments, the first core linker, second core linker, first capture linker, second capture linker, third capture linker independently comprise a reactive molecule or DNA sequence domain. In some embodiments, each reactive molecule independently comprises an amine, a thiol, a DBCO, a maleimide, biotin, an azide, an acrydite, a NHS-ester, a single stranded nucleic acid (RNA or DNA) of specific sequence, one or more polymers like PEG or polymerization initiators, or combinations thereof. In some embodiments, the linkage between the capture barcode and 1) the first core linker, and/or 2) the third capture linker comprises a chemical bond. In some embodiments, the chemical bond comprises a covalent bond. In some embodiments, for any method disclosed herein, the capture molecule is bound to the third capture linker through a chemical bond. In some embodiments, the capture molecule is covalently bonded to the third capture linker.

In some embodiments, for any method disclosed herein, each supramolecular DNA origami structure further comprises an anchor molecule linked to the core structure. In some embodiments, the anchor molecule is linked to the core structure via an anchor barcode, wherein the anchor barcode comprises a first anchor linker, a second anchor linker, and an anchor bridge disposed between the first and second anchor linkers, wherein the first anchor linker is bound to a third core linker that is bound to a second location on the core structure, wherein the anchor molecule is linked to the second anchor linker. In some embodiments, the anchor molecule comprises an amine, a thiol, a DBCO, a maleimide, biotin, an azide, an acrydite, a NHS-ester, a single stranded nucleic acid (RNA or DNA) of specific sequence, one or more polymers like PEG or polymerization initiators, or combinations thereof. In some embodiments, the anchor bridge comprises a polymer core. In some embodiments, the polymer core of the anchor bridge comprises a nucleic acid (DNA or RNA) of specific sequence or a polymer like PEG. In some embodiments, the second core linker, first anchor linker, second anchor linker, and anchor molecule independently comprise an anchor reactive molecule or DNA sequence domain. In some embodiments, each anchor reactive molecule independently comprises an amine, a thiol, a DBCO, a maleimide, biotin, an azide, an acrydite, a NHS-ester, a single stranded nucleic acid (RNA or DNA) of specific sequence, one or more polymers like PEG or polymerization initiators, or combinations thereof. In some embodiments, the anchor molecule is linked to the second anchor linker through a chemical bond. In some embodiments, the anchor molecule is covalently bonded to the second anchor linker.

In some embodiments, for any method disclosed herein, the signal comprises the capture barcode corresponding to a supramolecular DNA origami structure that shifted to an excited state. In some embodiments, any method disclosed herein, further comprising separating each capture barcode from a corresponding capture molecule for at least one supramolecular DNA origami structure that shifted to an excited state, such that the corresponding signal comprises the respective capture barcode which may be a nucleic acid-based sequence for detection of the analyte molecule bound to the respective capture molecule. In some embodiments, at least one separated capture barcodes are analyzed using genotyping, qPCR, sequencing, or combinations thereof. In some embodiments, a plurality of analyte molecules in the sample are detected simultaneously through multiplexing via one or more supramolecular DNA origami structures that shifted to an excited state. In some embodiments, for any method disclosed herein, the capture molecule for each supramolecular DNA origami structure is configured for binding to one or more specific types of analyte molecules.

In some embodiments, for any method comprising using a plurality of supramolecular DNA origami structures disclosed herein, each core structure of the plurality of supramolecular DNA origami structures are identical to each other. In some embodiments, each supramolecular DNA origami structure comprises a prescribed shape, size, molecular weight, or combinations thereof, so as to reduce or eliminate cross-reactions between a plurality of supramolecular DNA origami structures. In some embodiments, each supramolecular DNA origami structure comprises a plurality of capture molecules. In some embodiments, each supramolecular DNA origami structure comprises a prescribed stoichiometry of the capture molecules so as to reduce or eliminate cross-reactions between the plurality of supramolecular DNA origami structures.

In some embodiments, the plurality of supramolecular DNA origami structures are attached to one or more solid supports, one or more solid substrates, or combinations thereof. In some embodiments, each solid substrate of the one or more solid substrates comprises a planar substrate. In some embodiments, a plurality of supramolecular DNA origami structures are disposed on the planar substrate, wherein the planar substrate comprises a plurality of binding sites, wherein each binding site is configured to link with a corresponding supramolecular DNA origami structure. In some embodiments, the plurality of supramolecular DNA origami structures are configured to detect the same analyte molecule. In some embodiments, for any method comprising using a planar substrate, further comprising providing a plurality of signaling elements configured to link with the captured analyte molecules of at least one supramolecular DNA origami structure that shifted to the excited state (as described herein). In some embodiments, each signaling element comprises a fluorescent molecule or microbes, a fluorescent polymer, highly charged nanoparticles, or polymer. In some embodiments, at least one supramolecular DNA origami structure of the plurality of supramolecular DNA origami structures is configured to detect a different analyte molecule from the other supramolecular DNA origami structures. In some embodiments, for any method comprising using a planar substrate, further comprising barcoding each supramolecular DNA origami structure so as to identify the location of each supramolecular DNA origami structure on the planar substrate. In some embodiments, for any method comprising using a planar substrate, further comprising providing a plurality of signaling elements configured to link with the captured analyte molecules of at least one supramolecular DNA origami structure that shifted to the excited state. In some embodiments, each signaling element comprises a fluorescent molecule or microbead, a fluorescent polymer, highly charged nanoparticles or polymer.

In some embodiments, for any method disclosed herein, the sample comprises a biological particle or a biomolecule. In some embodiments, for any method disclosed herein, the sample comprises an aqueous solution comprising a protein, a peptide, a fragment of a peptide, a lipid, DNA, RNA, an organic molecule, a viral particle, an exosome, an organelle, or any complexes thereof. In some embodiments, for any method disclosed herein, the sample comprises a tissue biopsy, blood, blood plasma, Urine, Saliva, Tear, Cerebrospinal fluid, extracellular fluid, cultures cells, culture media, discarded tissue, plant matter, a synthetic protein, a bacterial and/or viral sample or fungal tissue, or combinations thereof.

Provided herein, in some embodiments, is a substrate for detecting one or more analyte molecules in a sample, the substrate comprising a plurality of supramolecular DNA origami structures, each supramolecular DNA origami structure comprising: a) a core structure comprising a plurality of core molecules, b) a capture molecule linked to the supramolecular core at a first location, wherein, upon recognition of an analyte molecule, the interaction triggers a respective supramolecular DNA origami structure to shift to an excited state and provide a signal for detecting the respective analyte molecule.

Provided herein, in some embodiments, is a substrate for detecting one or more analyte molecules in a sample, the substrate comprising a plurality of supramolecular structures, each supramolecular structure comprising: a) a core structure comprising a plurality of core molecules, b) a capture molecule linked to the supramolecular core at a first location, wherein, the capture molecule is configured to interact with a particular analyte molecule, such that the interaction triggers the respective supramolecular structure to shift to an excited state, so as to enable a signal to be generated for detecting the respective analyte molecule. In some embodiments, the supramolecular structure comprise a supramolecular DNA origami structure.

In some embodiments, the respective analyte molecule is 1) bound to the capture molecule through a chemical bond. In some embodiments, the capture molecule for each supramolecular DNA origami structure independently comprises a protein, a peptide, an antibody, an aptamer (RNA and DNA), a fluorophore, a darpin, a catalyst, a polymerization initiator, a polymer like PEG, or combinations thereof.

In some embodiments, the interaction between the respective analyte molecule and capture molecule comprises the respective analyte molecule forming a linkage with the capture molecule. In some embodiments, the linkage comprises a chemical bond. In some embodiments, the capture molecule for each supramolecular DNA origami structure independently comprises a protein, a peptide, an antibody, an aptamer (RNA and/or DNA), a fluorophore, a darpin, a catalyst, a polymerization initiator, a polymer like PEG, or combinations thereof. In some embodiments, the aptamer comprises a modified aptamer.

In some embodiments, the sample comprises a biological particle or a biomolecule. In some embodiments, the sample comprises an aqueous solution comprising a protein, a peptide, a fragment of a peptide, a lipid, DNA, RNA, an organic molecule, a viral particle, an exosome, an organelle, or any complexes thereof. In some embodiments, the sample comprises a tissue biopsy, blood, blood plasma, Urine, Saliva, Tear, Cerebrospinal fluid, extracellular fluid, cultures cells, culture media, discarded tissue, plant matter, a synthetic protein, a bacterial and/or viral sample or fungal tissue, or combinations thereof.

In some embodiments, the sample comprises a complex biological sample and the method provides for single-molecule sensitivity thereby increasing the dynamic range and enables quantitative capture of a range of molecular concentrations within the complex biological sample. In some embodiments, the analyte molecule comprises a protein, a peptide, a peptide fragment, a lipid, a DNA, a RNA, an organic molecule, an inorganic molecule, complexes thereof, or any combinations thereof. In some embodiments, the supramolecular DNA origami structure is a nanostructure. In some embodiments, the core structure is a nanostructure. In some embodiments, the plurality of core molecules for the core structure are arranged into a pre-defined shape and/or have a prescribed molecular weight. In some embodiments, the pre-defined shape is configured to limit or prevent cross-reactivity with another supramolecular DNA origami structure. In some embodiments, the plurality of core molecules for each core structure comprises one or more nucleic acid strands, one or more branched nucleic acids, one or more peptides, one or more small molecules, or combinations thereof. In some embodiments, the core structure independently comprises a scaffolded deoxyribonucleic acid (DNA) origami, a scaffolded ribonucleic acid (RNA) origami, a scaffolded hybrid DNA:RNA origami, a single-stranded DNA tile structure, a multi-stranded DNA tile structure, a single-stranded RNA origami, a multi-stranded RNA tile structure, hierarchically composed DNA or RNA origami with multiple scaffolds, a peptide structure, or combinations thereof.

## Claims

1. A system for detecting analyte molecules in a sample, comprising:
(a) a substrate comprising a plurality of binding locations, each configured to receive one of the supramolecular structures;
(b) a plurality of supramolecular structures, wherein each supramolecular structure comprises:
i. at least one capture molecule configured to bind to an analyte molecule,
ii. a core structure comprising a plurality of core molecules, wherein the at least one capture molecule is linked to the core structure through a capture barcode, and
iii. the capture barcode linked to the core structure at a first location, that can be used to map the location of each respective supramolecular structure on the substrate;
(c) the sample comprising the one or more analytes, wherein upon contacting the sample with the substrate, the one or more analyte molecules interact with a corresponding capture molecule of the plurality of capture molecules;
(d) a signal generation system configured to generate a signal for each analyte molecule bound to a corresponding capture molecule; and
(e) a detection system configured to detect the generated signal;
wherein the locations of the supramolecular structures received on the plurality of binding locations are configured to be mapped using the capture barcodes prior to contacting the supramolecular structures with the sample, thereby allowing creation of a map of unique supramolecular structure locations on the substrate.

2. The system of claim 1, wherein each capture barcode is configured to link with one of the capture molecules.

3. The system of claim 1, wherein the capture molecules are configured to interact with different types of analyte molecules.

4. The system of claim 1, wherein the capture molecules are each configured to interact with a particular analyte molecule.

5. The system of claim 1, wherein the supramolecular structures are DNA origami structures, and the capture molecules are attached to the DNA origami structures through DNA hybridization.

6. The system of claim 5, wherein the supramolecular structure comprises a core structure comprising a scaffolded DNA origami structure.

7. A method for detecting analyte molecules present in a sample, the method comprising:
providing a plurality of supramolecular structures bound to a substrate at binding locations, each supramolecular structure comprising:
i. at least one capture molecule configured to bind to an analyte molecule;
ii. a core structure comprising a plurality of core molecules, wherein the at least one capture molecule is linked to the core structure through a capture barcode, and
iii. the capture barcode linked to the core structure at a first location;
mapping the location of the plurality of supramolecular structures on the substrate prior to contacting the supramolecular structures with the sample and thereby creating a map of unique supramolecular structure locations on the substrate;
contacting the plurality of supramolecular structures with the sample, such that one or more capture molecules of the plurality of supramolecular structures interacts with a corresponding analyte molecule of the one or more analyte molecules;
generating a signal for each analyte molecule bound to a corresponding capture molecule; and
detecting the signals and thereby detecting the analyte molecules present in the sample;
wherein a capture barcode is linked with each supramolecular structure, and the supramolecular structures on the substrate are mapped via the capture barcodes.

8. The method of claim 7, wherein each capture barcode is configured to link with one of the capture molecules.

9. The method of claim 7, wherein mapping the supramolecular structures on the substrate is performed when the substrate is manufactured.

10. The method of claim 7, further comprising immobilizing the capture molecules on the substrate, and wherein mapping the supramolecular structures on the substrate is performed after the capture molecules have been immobilized on the substrate.

11. The method of claim 7, wherein the capture molecules are configured to interact with different types of analyte molecules.

12. The method of claim 7, wherein the capture molecules are each configured to interact with the same type of analyte molecule.

13. The method of claim 7, further comprising quantifying concentrations of the analyte molecules present in the sample through registration of the mapped capture molecule locations with the detected signals.

14. The method of claim 7, wherein the supramolecular structures comprises a core structure comprising a scaffolded DNA origami structures, and the capture molecules are attached to the DNA origami structures through DNA hybridization.

## Patentansprüche

1. System zum Nachweisen von Analytmolekülen in einer Probe, umfassend:
(a) ein Substrat, das eine Vielzahl von Bindungsstellen umfasst, die jeweils dazu konfiguriert sind, eine der supramolekularen Strukturen aufzunehmen;
(b) eine Vielzahl von supramolekularen Strukturen, wobei jede supramolekulare Struktur Folgendes umfasst:
i. mindestens ein Erfassungsmolekül, das dazu konfiguriert ist, an ein Analytmolekül zu binden,
ii. eine Kernstruktur, die eine Vielzahl von Kernmolekülen umfasst, wobei das mindestens eine Erfassungsmolekül durch einen Erfassungsbarcode mit der Kernstruktur verknüpft ist, und
iii. den Erfassungsbarcode, der mit der Kernstruktur an einer ersten Position verknüpft ist, der verwendet werden kann, um die Position jeder jeweiligen supramolekularen Struktur auf dem Substrat abzubilden;
(c) die Probe, die den einen oder die mehreren Analyten umfasst, wobei beim Kontaktieren der Probe mit dem Substrat das eine oder die mehreren Analytmoleküle mit einem entsprechenden Erfassungsmolekül der Vielzahl von Erfassungsmolekülen interagieren;
(d) ein Signalerzeugungssystem, das dazu konfiguriert ist, ein Signal für jedes Analytmolekül zu erzeugen, das an ein entsprechendes Erfassungsmolekül gebunden ist; und
(e) ein Nachweissystem, das dazu konfiguriert ist, das erzeugte Signal nachzuweisen;
wobei die Positionen der supramolekularen Strukturen, die an der Vielzahl von Bindungsstellen aufgenommen werden, dazu konfiguriert sind, unter Verwendung der Erfassungsbarcodes abgebildet zu werden, bevor die supramolekularen Strukturen mit der Probe in Kontakt gebracht werden, wodurch die Erstellung einer Karte von einzigartigen supramolekularen Strukturpositionen auf dem Substrat ermöglicht wird.

2. System nach Anspruch 1, wobei jeder Erfassungsbarcode dazu konfiguriert ist, sich mit einem der Erfassungsmoleküle zu verknüpfen.

3. System nach Anspruch 1, wobei die Erfassungsmoleküle dazu konfiguriert sind, mit verschiedenen Arten von Analytmolekülen zu interagieren.

4. System nach Anspruch 1, wobei die Erfassungsmoleküle jeweils dazu konfiguriert sind, mit einem bestimmten Analytmolekül zu interagieren.

5. System nach Anspruch 1, wobei die supramolekularen Strukturen DNA-Origami-Strukturen sind und die Erfassungsmoleküle durch DNA-Hybridisierung an den DNA-Origami-Strukturen befestigt sind.

6. System nach Anspruch 5, wobei die supramolekulare Struktur eine Kernstruktur umfasst, die eine gerüstbasierte DNA-Origami-Struktur umfasst.

7. Verfahren zum Nachweisen von Analytmolekülen, die in einer Probe vorhanden sind, wobei das Verfahren Folgendes umfasst:
Bereitstellen einer Vielzahl von supramolekularen Strukturen, die an Bindungsstellen an ein Substrat gebunden sind, wobei jede supramolekulare Struktur Folgendes umfasst:
i. mindestens ein Erfassungsmolekül, das dazu konfiguriert ist, an ein Analytmolekül zu binden;
ii. eine Kernstruktur, die eine Vielzahl von Kernmolekülen umfasst, wobei das mindestens eine Erfassungsmolekül durch einen Erfassungsbarcode mit der Kernstruktur verknüpft ist, und
iii. den Erfassungsbarcode, der mit der Kernstruktur an einer ersten Position verknüpft ist;
Abbilden der Position der Vielzahl von supramolekularen Strukturen auf dem Substrat vor dem In-Kontaktbringen der supramolekularen Strukturen mit der Probe und dadurch Erstellen einer Karte von einzigartigen supramolekularen Strukturpositionen auf dem Substrat;
In-Kontaktbringen der Vielzahl von supramolekularen Strukturen mit der Probe, sodass ein oder mehrere Erfassungsmoleküle der Vielzahl von supramolekularen Strukturen mit einem entsprechenden Analytmolekül des einen oder der mehreren Analytmoleküle interagieren;
Erzeugen eines Signals für jedes Analytmolekül, das an ein entsprechendes Erfassungsmolekül gebunden ist; und Nachweisen der Signale und dadurch Nachweisen der Analytmoleküle, die in der Probe vorhanden sind;
wobei ein Erfassungsbarcode mit jeder supramolekularen Struktur verknüpft ist und die supramolekularen Strukturen auf dem Substrat über die Erfassungsbarcodes abgebildet werden.

8. Verfahren nach Anspruch 7, wobei jeder Erfassungsbarcode dazu konfiguriert ist, sich mit einem der Erfassungsmoleküle zu verknüpfen.

9. Verfahren nach Anspruch 7, wobei das Abbilden der supramolekularen Strukturen auf dem Substrat durchgeführt wird, wenn das Substrat hergestellt wird.

10. Verfahren nach Anspruch 7, ferner umfassend das Immobilisieren der Erfassungsmoleküle auf dem Substrat, und wobei das Abbilden der supramolekularen Strukturen auf dem Substrat durchgeführt wird, nachdem die Erfassungsmoleküle auf dem Substrat immobilisiert worden sind.

11. Verfahren nach Anspruch 7, wobei die Erfassungsmoleküle dazu konfiguriert sind, mit verschiedenen Arten von Analytmolekülen zu interagieren.

12. Verfahren nach Anspruch 7, wobei die Erfassungsmoleküle jeweils dazu konfiguriert sind, mit der gleichen Art von Analytmolekül zu interagieren.

13. Verfahren nach Anspruch 7, ferner umfassend das Quantifizieren von Konzentrationen der Analytmoleküle, die in der Probe vorhanden sind, durch Registrierung der abgebildeten Erffassungsmolekülpositionen mit den nachgewiesenen Signalen.

14. Verfahren nach Anspruch 7, wobei die supramolekularen Strukturen eine Kernstruktur umfassen, die gerüstbasierte DNA-Origami-Strukturen umfasst, und die Erfassungsmoleküle durch DNA-Hybridisierung an den DNA-Origami-Strukturen befestigt sind.

## Revendications

1. Système destiné à la détection des molécules d'analyte dans un échantillon, comprenant :
(a) un substrat comprenant une pluralité d'emplacements de liaison, chacun étant conçu pour recevoir l'une des structures supramoléculaires ;
(b) une pluralité de structures supramoléculaires, chaque structure supramoléculaire comprenant :
i. au moins une molécule de capture conçue pour se lier à une molécule d'analyte,
ii. une structure de cœur comprenant une pluralité de molécules de cœur, ladite au moins une molécule de capture étant liée à la structure de cœur par l'intermédiaire d'un code-barres de capture, et
iii. le code-barres de capture lié à la structure de cœur au niveau d'un premier emplacement, pouvant être utilisé pour cartographier l'emplacement de chaque structure supramoléculaire respective sur le substrat ;
(c) l'échantillon comprenant le ou les analytes, lors de la mise en contact de l'échantillon avec le substrat, une ou plusieurs molécules d'analyte interagissant avec une molécule de capture correspondante de la pluralité de molécules de capture ;
(d) un système de génération de signal conçu pour générer un signal pour chaque molécule d'analyte liée à une molécule de capture correspondante ; et
(e) un système de détection conçu pour détecter le signal généré ;
lesdits emplacements des structures supramoléculaires reçues sur la pluralité d'emplacements de liaison étant conçus pour être cartographiés à l'aide des codes-barres de capture avant la mise en contact des structures supramoléculaires avec l'échantillon, permettant ainsi la création d'une carte d'emplacements uniques de structures supramoléculaires sur le substrat.

2. Système de la revendication 1, chaque code-barres de capture étant conçu pour se lier à l'une des molécules de capture.

3. Système de la revendication 1, lesdites molécules de capture étant conçues pour interagir avec différents types de molécules d'analyte.

4. Système de la revendication 1, lesdites molécules de capture étant chacune conçues pour interagir avec une molécule d'analyte particulière.

5. Système de la revendication 1, lesdites structures supramoléculaires étant des structures d'origami d'ADN, et lesdites molécules de capture étant fixées aux structures d'origami d'ADN par hybridation d'ADN.

6. Système de la revendication 5, ladite structure supramoléculaire comprenant une structure de cœur comprenant une structure d'origami d'ADN échafaudée.

7. Procédé permettant la détection de molécules d'analyte présentes dans un échantillon, le procédé comprenant :
la fourniture d'une pluralité de structures supramoléculaires liées à un substrat au niveau d'emplacements de liaison, chaque structure supramoléculaire comprenant :
i. au moins une molécule de capture conçue pour se lier à une molécule d'analyte ;
ii. une structure de cœur comprenant une pluralité de molécules de cœur, ladite au moins une molécule de capture étant liée à la structure de cœur par l'intermédiaire d'un code-barres de capture, et
iii. le code-barres de capture lié à la structure de cœur au niveau d'un premier emplacement ;
la cartographie de l'emplacement de la pluralité de structures supramoléculaires sur le substrat avant la mise en contact des structures supramoléculaires avec l'échantillon et, ainsi, la création d'une carte d'emplacements uniques de structures supramoléculaires sur le substrat ;
la mise en contact de la pluralité de structures supramoléculaires avec l'échantillon, de sorte qu'une ou plusieurs molécules de capture de la pluralité de structures supramoléculaires interagissent avec une molécule d'analyte correspondante de la ou des molécules d'analyte ;
la génération d'un signal pour chaque molécule d'analyte liée à une molécule de capture correspondante ; et la détection des signaux et, ainsi, la détection des molécules d'analyte présentes dans l'échantillon ;
un code-barres de capture étant lié à chaque structure supramoléculaire, et lesdites structures supramoléculaires sur le substrat étant cartographiées par l'intermédiaire des codes-barres de capture.

8. Procédé de la revendication 7, chaque code-barres de capture étant conçu pour se lier à l'une des molécules de capture.

9. Procédé de la revendication 7, ladite cartographie des structures supramoléculaires sur le substrat étant réalisée lorsque le substrat est fabriqué.

10. Procédé de la revendication 7, comprenant en outre l'immobilisation des molécules de capture sur le substrat, et la cartographie desdites structures supramoléculaires sur le substrat est réalisée après que les molécules de capture ont été immobilisées sur le substrat.

11. Procédé de la revendication 7, lesdites molécules de capture étant conçues pour interagir avec différents types de molécules d'analyte.

12. Procédé de la revendication 7, lesdites molécules de capture étant chacune conçues pour interagir avec le même type de molécule d'analyte.

13. Procédé de la revendication 7, comprenant en outre la quantification de concentrations des molécules d'analyte présentes dans l'échantillon par enregistrement des emplacements de molécules de capture cartographiés avec les signaux détectés.

14. Procédé de la revendication 7, lesdites structures supramoléculaires comprenant une structure de cœur comprenant des structures d'origami d'ADN échafaudées, et lesdites molécules de capture étant fixées aux structures d'origami d'ADN par hybridation d'ADN.
